(19) Europäisches Patentamt / European Patent Office / Office européen des brevets

(11) **EP 1 576 184 B1**

(12) **EUROPÄISCHE PATENTSCHRIFT**

(45) Veröffentlichungstag und Bekanntmachung des Hinweises auf die Patenterteilung:
**23.05.2007 Patentblatt 2007/21**

(21) Anmeldenummer: **02799063.9**

(22) Anmeldetag: **19.12.2002**

(51) Int Cl.:
**C12Q 1/68** [(2006.01)]

(86) Internationale Anmeldenummer:
**PCT/EP2002/014584**

(87) Internationale Veröffentlichungsnummer:
**WO 2003/056034 (10.07.2003 Gazette 2003/28)**

(54) **VERFAHREN ZUR IN-VITRO SELEKTION VON BINDERN**

METHOD FOR THE IN VITRO SELECTION OF BINDERS

PROCEDE DE SELECTION IN VITRO DE LIANTS

(84) Benannte Vertragsstaaten:
**AT BE BG CH CY CZ DE DK EE ES FI FR GB GR IE IT LI LU MC NL PT SE SI SK TR**

(30) Priorität: **24.12.2001 DE 10163986**
**09.02.2002 DE 10205423**
**11.02.2002 DE 10205571**

(43) Veröffentlichungstag der Anmeldung:
**21.09.2005 Patentblatt 2005/38**

(73) Patentinhaber: **Arrowhead Research Corporation Pasadena, CA 91101 (US)**

(72) Erfinder:
• **STEIPE, Boris**
**Toronto, Ontario M6G 3K3 (CA)**
• **CLAUSEN-SCHAUMANN, Hauke**
**82340 Feldafing (DE)**
• **OESTERHELT, Filipp**
**80339 München (DE)**

(74) Vertreter: **Eisenführ, Speiser & Partner Patentanwälte Rechtsanwälte,**
**Postfach 31 02 60**
**80102 München (DE)**

(56) Entgegenhaltungen:
**WO-A-00/57756       WO-A-02/14862**
**WO-A-99/45142       US-A- 5 445 970**
**US-B1- 6 180 418**

• **FLORIN E-L ET AL: "ADHESION FORCES BETWEEN INDIVIDUAL LIGAND-RECEPTOR PAIRS" SCIENCE, AMERICAN ASSOCIATION FOR THE ADVANCEMENT OF SCIENCE,, US, Bd. 264, 15. April 1994 (1994-04-15), Seiten 415-417, XP002072145 ISSN: 0036-8075**

## Beschreibung

Die Erfindung betrifft ein Verfahren zur Auswahl von mit einem Zielmolekül bindefähigen Bindern unter Ausnutzung eines differentiellen Krafttests.

In vielen Bereichen der Biochemie und Medizin sind Moleküle bekannt, die andere Moleküle spezifisch binden können. Für derartige Bindungspaare sind viele interessante Anwendungen beschrieben worden, die unter anderem in den Bereichen Analytik, Diagnostik und Therapie nützlich sind. So erlaubt die Entwicklung und Untersuchung von spezifisch bindefähigen Molekülen Rückschlüsse auf Metabolismen und den Ablauf biochemischer Reaktionskaskaden im Bereich der Forschung und der Diagnostik, oder schafft Möglichkeiten zur Beeinflussung des Bindungspartners durch Aktivierung oder Hemmung im Bereich der Therapie. Eine wichtige Aufgabe in Therapie und Diagnostik ist daher die Untersuchung von spezifisch bindefähigen Paaren und das Auffinden von spezifischen Bindern für bekannte Zielmoleküle oder von Bindern, deren Zielmoleküle zwar noch nicht bekannt sind, bei denen das Bindungsereignis aber eine Auswirkung auf Zellen oder Organismen hat, geworden. Da der Entwurf solcher Binder allein aus Kenntnis der Prinzipien von Physik und Chemie im allgemeinen nicht gelingt, sind Methoden entwickelt worden, die in Anlehnung an das natürliche Prinzip der Evolution durch Bereitstellung einer Vielfalt von Molekülen und Selektion solcher mit gewünschten Eigenschaften zu geeigneten Bindern gelangen. Hierzu sind Selektionsverfahren notwendig, um z.B. spezifisch bindende Moleküle aus einem Gemisch chemisch ähnlicher Moleküle mit unterschiedlichen Bindungseigenschaften, das also aus einer Vielzahl möglicher Bindekandidaten besteht, aufzufinden. Ziel ist es meist, solche Moleküle aufzufinden, die ein hohes Maß an Spezifität und/oder Affinität für ein Zielmolekül haben. Die Erfolgschancen solcher Verfahren werden im allgemeinen durch zwei Kriterien bestimmt: zum einen die Komplexität des verwendeten Gemischs - geeignete Binder können nur aus einem Gemisch ausgewählt werden, in dem sie tatsächlich enthalten sind - zum anderen das Selektionsverfahren, das möglichst gut auf die gewünschten Eigenschaften des Binders abgestimmt sein soll.

Eine häufig gewünschte Eigenschaft von Bindern ist - unter anderem - spezifische Bindefähigkeit. Spezifisch bindefähige Moleküle sind interessant für Forschung und Diagnostik, um spezifisch Komponenten in geringer Konzentration nachzuweisen. In der Diagnostik werden z.B. schon lange Antikörper verwendet, die Antigene spezifisch erkennen. Antikörper müssen jedoch aufwendig durch Immunisierung von Tieren und Hybridomabildung gewonnen werden.

Spezifisch bindefähige Moleküle sind auch interessant für die Therapie, wo sie beispielsweise als Pharmaka durch Aktivierung oder Hemmung von Zielmolekülen wirken.

Erwünscht ist es daher, spezifisch bindefähige Substanzen aufzufinden, die sich in vivo oder in vitro vermehren lassen und dann in der Diagnostik, zum Nachweis von Substanzen und in der Therapeutik, z.B. um Rezeptoren aufzufinden, zu blockieren oder zu aktivieren, eingesetzt werden können. Die zu untersuchende spezifische Bindung zwischen Molekülen beruht in der Mehrzahl der Fälle nicht auf kovalenten Wechselwirkungen, sondern auf der Interaktion von Bindungspartnern durch Wasserstoffbrücken, ionische, hydrophobe und van-der-Waals-Kräfte. Diese Wechselwirkungen sind um Größenordnungen schwächer als kovalente Bindungen, die durch chemische Reaktionen gebildet oder gelöst werden, und sind daher auch durch äußere Einflüsse leichter beeinflußbar. Nicht kovalente Wechselwirkungen zwischen Bindungspartnern mit einem hohen Grad an selektiver Bindeeigenschaft sind die Voraussetzung für die molekulare Erkennung.

Eine häufige Aufgabenstellung bei der Entwicklung von Therapeutika und Diagnostika besteht folglich darin, zu einem bestimmten Zielmolekül einen passenden Bindepartner (Binder) zu finden. Die Wechselwirkung zwischen Binder und Zielmolekül soll typischerweise ein hohes Maß an Spezifität und/oder Affinität aufweisen, welche auf einer molekularen Erkennung beruhen.

Um derartige Interaktionen ausfindig zu machen, bedient man sich i.d.R. heterogener Bindetests, also Bindetests, bei denen das Zielmolekül auf einer Festphase gebunden und mit den Binderkandidaten inkubiert wird. Zunächst finden Bindungsereignisse statt. Nach einem Trennschritt, bei dem die nicht gebundenen Binderkandidaten von der Festphase abgespült werden, können nun die an die Zielmoleküle gebundenen Binder nachgewiesen werden.

Zu Molekülen, die als Binder wirken, gehören u.a. neben den natürlich vorkommenden Immunglobulinen (Antikörpern) zahlreiche rekombinante bzw. synthetische Binder, darunter Antikörper-Fragmente, wie single chain fragments (scFvs) oder auf Nukleinsäuren basierende Aptamere.

Eine klassische Methode zur Gewinnung biologischer Binder in Form von Antikörpern ist die Immunisierung, bei der man sich die Immunantwort eines Tieres (polyklonale Antikörper) oder einer Zellkultur (monoklonale Antikörper) gegenüber einem Antigen zu Nutze macht.

Die geläufigste Vorgehensweise, um zu einem bestimmten Zielmolekül den passenden Binder durch einen *in vitro* Bindetest ausfindig zu machen, ist das Screening. Beim herkömmlichen Screening werden die verschiedenen Binderkandidaten in getrennten Ansätzen zu dem Zielmolekül gegeben. Da es nur sehr selten vorkommt, dass zwischen einem Binder und einem Zielmolekül eine zufällige spezifische Interaktion auftritt, verwendet man beim Screening Sammlungen mit einer sehr großen Anzahl von Substanzen, die typischerweise (aber nicht notwendigerweise) chemisch ähnlich sind, sich jedoch in bestimmten Bereichen ihres molekularen Aufbaus unterscheiden, sogenannte Bibliotheken.

In der Regel erstellt man mit kombinatorischen Verfahren Substanzbibliotheken, die z.B. bis zu $10^{15}$ unter-

schiedliche Mitglieder oder auch mehr enthalten können. Daher mussten Verfahren entwickelt werden, um die Bindefähigkeit einer sehr großen Zahl von Substanzen parallel für vorgesehene Zielmoleküle testen zu können. Um solch eine Bibliothek "Molekül für Molekül" auf Interaktion zum Zielmolekül zu screenen, bedarf es also sehr vieler getrennter Testansätze, was einen beträchtlichen Aufwand bedeutet.

Eine Alternative zum herkömmlichen Screening wurde durch die Verwendung biologischer Bindermoleküle möglich, die mit ihrer genetischen Information verknüpft und somit vermehrbar (amplifizierbar) sind. Statt eines Screens mit vielen Ansätzen führt man eine sogenannte in-vitro-Selektion durch, bei dem eine gesamte Bibliothek von Bindern in einem Ansatz mit dem Zielmolekül inkubiert wird.

Dies bedeutet gegenüber dem Screening einen sehr viel höheren Durchsatz und entsprechend geringeren Aufwand. Um heraus zu finden, welche der vielen verschiedenen (bis zu $10^{15}$ oder mehr) Binderkandidaten gebunden haben, werden diese amplifiziert, da die auf der Oberfläche verbleibenden Mengen so klein sind, dass sie in der Regel nicht durch chemische Analytik identifiziert werden können.

Um möglichst spezifische und/oder hoch affine Binder für ein Zielmolekül aufzufinden, kann man, wie oben erwähnt, eine Auswahl aus einer Vielzahl von Molekülen experimentell durchführen. In der Regel erstellt man, wie erwähnt, Substanzbibliotheken, die z.B. bis zu $10^{15}$ unterschiedliche Mitglieder oder auch mehr enthalten können. Daher mussten Verfahren entwickelt werden, um die Bindefähigkeit einer sehr großen Zahl von Substanzen parallel für vorgegebene Zielmoleküle testen zu können.

Ein Zyklus eines in vitro Selektionsexperiments - so wie dies dem Fachmann bekannt ist - besteht in der Regel aus folgenden Schritten: Die Binderbibliothek wird mit dem Zielmolekül in Kontakt gebracht. Die Binder, die im Verhältnis zur Bibliothek eine höhere Affinität aufweisen, werden von den übrigen Molekülen der Bibliothek abgetrennt. Dies kann beispielsweise durch Wegwaschen der niedrigaffineren Moleküle erfolgen. Die verbleibenden Binder werden isoliert und anschließend amplifiziert. In der Regel muss dieser Selektionszyklus zur Anreicherung hochaffiner Binder mehrmals wiederholt werden, da auch niedrig-affine und/oder unspezifisch bindende Moleküle von Runde zu Runde mitgeschleppt werden können.

Für die Selektion von Peptidbindern wurden unterschiedliche Verfahren entwickelt. Grundsätzlich ist es hierbei notwendig, die amplifizierbare genetische Information, die für die Aminosäuresequenz des Peptids kodiert, mit dem nicht-amplifizierbaren Peptid zu verknüpfen. Dies kann durch die Präsentation der Peptide auf der Oberfläche von Phagen und Bakterien oder durch die physische Kopplung des genetischen Trägers, der RNA oder DNA, mit dem Peptid selbst erreicht werden.

Hierzu sind bereits einige Verfahren bekannt, die zur in-vitro-Selektion geeignet sind.

Für das Screenen von antigenspezifischen Antikörperfragmenten ist z.B. das so genannte "PANNING" von Phage-Display Bibliotheken bekannt. Hier werden mit Antigen beschichtete Oberflächen mit einer Antikörper-Phagmid Bibliothek in Kontakt gebracht, die an Antigen gebundenen Antikörper werden von ungebundenen Antikörpern getrennt und anschließend werden die Phagmide in vivo repliziert, wodurch die Antikörperfragmente zur weiteren Verwendung hergestellt und in ihrer Sequenz und ihren Eigenschaften charakterisiert werden können.

Phage-Display-Bibliotheken können durch Insertion von randomisierten oder teilweise randomisierten DNA-Fragmenten in die Gene der Coat-Proteine pIII oder pVIII von filamentösen Bakteriophagen hergestellt werden. Die Technik von Phage-Display-Selektionen ist beispielsweise in Methods in Enzymology, Vol. 267, 1996, Academic Press, Inc., S.3-109 beschrieben. Die Peptidbibliotheken werden dabei auf der Oberfläche der Bakteriophagen präsentiert. Die Bibliothek aus Bakteriophagen wird dann mit dem immobilisierten Zielmolekül in Kontakt gebracht und alle nicht gebundenen Phagen entfernt. Die Rückgewinnung der gebundenen Phagen kann beispielsweise unter sauren Bedingungen (pH 2.2) erfolgen. Die Amplifikation der hochaffinen Peptide erfolgt in der Selektion durch die Infektion von Bakterien und die Vermehrung der Phagen. Beschriebene Nachteile des Phage displays sind beispielsweise die Anreicherung von unspezifischen Bindern, oder die Anreicherung von Proteinen, die zwar nicht spezifisch das Zielmolekül binden, aber die sich wesentlich besser exprimieren lassen und deswegen den Phagen einen Selektionsvorteil verleihen.

Eine Limitation der Technologien, die zur Vermehrung der genetischen Information lebende Systeme wie eukaryontische Zellen, Bakterien und Bakteriophagen verwenden, ist die begrenzte Transformationseffizienz, die die Komplexität der verwendeten Bibliotheken auf maximal ca. $10^9$ verschiedene Moleküle begrenzt. Des weiteren ist die Kultur und Vermehrung der Zellen sehr zeitaufwändig. Daher wurden zellfreie in vitro Systeme, wie das Ribosomen-Display entwickelt (Hanes und Plückthun, A. (1997), Proc. Natl. Acad. Sci. USA 91, 4937-4942; Hanes et al. (1998) Proc. Natl. Acad. Sci. USA 95, 14130-14135). Die für die Peptidbibliothek codierende DNA wird in mRNA transkribiert und in in vitro Translationsreaktionen in die Proteine übersetzt. Durch Abkühlen auf 4°C wird die Translation gestoppt. Die Komplexe aus mRNA, Ribosom und Polypeptid bleiben so intakt, die genetische Information (mRNA) bleibt also mit dem Polypeptid verbunden. Die Komplexe werden mit dem immobilisierten Zielmolekül inkubiert und alle nicht bindenden Komplexe weggewaschen. Durch Denaturierung kann dann die mRNA, die für ein Peptid kodiert, das das Zielmolekül bindet, wiedergewonnen werden und durch reverse Transkription und PCR amplifiziert werden. Danach kann ein neuer Selektionszyklus begonnen werden. Ein Nachteil des Ribosome display ist es beispielsweise, daß die Selektion unter reduzierenden, genau kontrollierten Bedingungen durchgeführt werden muss. Eine Aufgabe der Erfindung ist es demnach, ein Verfahren zur Verfügung zu stellen, das robust gegenüber den Pufferbedingungen ist, in denen es durchgeführt wird

Eine weitere Methode zur physikalischen Verbindung der RNA mit dem Peptid wurde von Roberts und Szostak entwickelt (Roberts, R.W. und Szostak, J.W., 1997, Proc. Natl. A-cad. Sci. 94, S. 12297-12302). Hierbei wird die für die Peptidbibliothek kodierende DNA in vitro in RNA umgeschrieben. An die RNA wird dann ein synthetischer DNA-Linker ligiert, der an seinem 3'-Ende ein Puromycin-Molekül trägt. Die RNA wird in einer in vitro Translationsreaktion in die Peptide umgeschrieben. Sobald das Ribosom das Ende des RNA-Moleküls erreicht, stoppt die Translation an der Verbindung der RNA mit dem DNA-Linker. Das Puromycin gelangt in das Ribosom und wird kovalent mit dem Peptid verknüpft. Das Hybridmolekül aus Peptid und Nukleinsäure kann dann vom Rest des Ribosoms getrennt und aufgereinigt werden. Nach der Selektion werden die am Zielmolekül gebundenen Fusionsmoleküle isoliert und die RNA wird durch reverse Transkription und PCR amplifiziert und ein neuer Selektionszyklus gestartet. Ein Vorteil der Methode ist, dass die Fusionsmoleküle auch unter denaturierenden Bedingungen gewaschen werden können, und nicht wie beim Phage-Display oder dem Ribosomen-Display auf die Intaktheit der Komplexe (Phagen oder Ribosomen) geachtet werden muss. Dies ermöglicht die Anwendung stringenterer Bedingungen während der Selektion, um affinere Moleküle zu evolvieren.

Andere, aber nicht limitierende Beispiele für Affinitätsselektionen sind das Bakterien-Display (Georgiou G. et al., (1993), Trends Biotechnol. 11, 6-10), Polysomen-Display (Mattheakis, L. C. et al. (1994), Proc. Natl. Acad. Sci. 91, 9022-9026) oder Lac-Repressor-Fusionsproteine (Gates C.M., (1996), J. Mol. Biol. 255, 373-386).

Im Jahr 1990 wurde eine in vitro Selektionstechnik entwickelt, die es erlaubt, hochaffine Nukleinsäureliganden, sogenannte Aptamere (Ellington A. und Szostak J. W., 1990, Nature 346, 818-822) zu erzeugen. Wie Proteine sind einzelsträngige Nukleinsäuren in der Lage, Sekundär- und Tertiärstrukturen auszubilden und so spezifische Bindungstaschen für Zielmoleküle auszubilden. Diese Technik der in vitro Selektion wird manchmal auch als SELEX ("systematic evolution of ligands by exponential enrichment") bezeichnet (Tuerk C. und Gold L.. 1990, Science 249, 505-510). Eine Beschreibung der Technologie findet sich auch in dem Europäischen Patent EP 0 533 838. Die Bibliotheken bestehen in der Regel aus synthetischen Nukleinsäuren, bei denen randomisierte Bereiche zwischen 20 und 120 Nukleotiden von konservierten Sequenzabschnitten flankiert werden, die als Primerannealingstellen zur Amplifikation der Moleküle dienen. Im Fall von RNA-Selektionen trägt einer der konservierten Bereiche zusätzlich einen Promotor für eine RNA-Polymerase, der es ermöglicht, das DNA-Templat in RNA zu transkribieren. Die Amplifikation der RNA kann durch reverse Transkription und PCR erreicht werden.

Die Nukleinsäurebibliotheken erreichen Komplexitäten von bis zu $10^{16}$ verschiedenen Molekülen. In einem RNA-Selektionsexperiment wird die initiale DNA-Bibliothek in RNA umgeschrieben. Danach werden die Ribonukleinsäuren mit dem Zielmolekül inkubiert. und die niedriger affinen Binder abgetrennt. Die Trennung kann zum Beispiel durch Affinitätschromatographie oder Retention der Komplexe auf Filtermembranen erfolgen.

Nach dem Selektionsexperiment können die RNA-Binder von dem Zielmolekül isoliert werden und über Standardreaktionen (reverse Transkription und PCR) wieder amplifiziert werden. Typischerweise müssen in einem in vitro Selektionsexperiment sechs bis zwanzig Selektionszyklen durchlaufen werden, um höher affine Nukleinsäuren anzureichern. Die Identifizierung der monoklonalen Sequenzen (Binder) erfolgt dann durch Klonierung und Sequenzierung.

Die aus ssDNA oder ssRNA bestehenden Aptamere besitzen sehr hohe Affinitäten und Spezifitäten für ihre Liganden. Bislang wurde eine Vielzahl dieser Oligonucleotide für kleine, organische Verbindungen, Peptide, Proteine, oder sogar komplexe Strukturen wie Viren und Zellen isoliert. Beschreibungen finden sich in folgenden Übersichtsartikeln: Gold L., Polisky B., Uhlenbeck O., Yarus M. (1995), Annu Rev Biochem, 64, 763-797), Ellington, A. D. and Conrad, R. (1995). Biotechnol Annu Rev, 1, 185-214; Famulok, M. (1999). Curr Opin Struct Biol, 9, 324-329.

Obwohl das Selex-Verfahren gegenüber früher benutzten Verfahren, bei denen z.B. Affinitätschromatographiestufen zwischengeschaltet werden mussten, eine Verbesserung darstellt, hat es doch auch einige Nachteile. Das Verfahren soll so durchgeführt werden, dass pro Inkubationsrunde etwa 3 bis 7% aller Nucleinsäuren an dem Zielmolekül gebunden werden. Hierdurch soll eine 10- bis 20-fache Anreicherung in jeder Runde erzielt werden. Da große, komplexe Substanzbibliotheken typischerweise nur wenige Moleküle mit den gesuchten Eigenschaften enthalten, werden mehrere Zyklen von Bindung, Selektion und Amplifikation notwendig, um diese in ausreichender Reinheit für die Analyse ihrer Zusammensetzung zu erhalten. Außerdem handelt es sich bei dem Selex-Verfahren um ein Verfahren zur Selektion von RNA- und DNA-Aptameren, die aufgrund des Aufbaus des Moleküls sehr bindefreudig sind. Um daher nicht zu viele unspezifisch gebundene Moleküle mitzuselektieren, müssen die Bedingungen der Inkubation sehr stringent eingestellt werden, damit nur die affineren Moleküle haften bleiben und eine zu starke Beladung der Zielmoleküle mit weniger affin gebundenen Molekülen vermieden wird. Dieses Verfahren ist daher nicht so geeignet, wenn die Bindefähigkeit unter physiologischen Bedingungen untersucht werden soll oder Bindemoleküle gesucht werden, die unter physiologischen Bedingungen eine hohe Spezifität und/oder Affinität zeigen.

Ein weiterer Nachteil bekannter Verfahren besteht darin, dass mehrere bis viele Runden der Inkubation und Amplifikation notwendig sind, bis eine Anreicherung von Molekülen mit gewünschten Eigenschaften erfolgt ist. Je stärker affin die Binder bereits sind, desto schwieriger wird es, Binder mit weiter gesteigerter Affinität sicher zu unterscheiden. Dies liegt daran, daß Affinität sich aus dem Quotienten der Bindungsrate und der Dissoziationsrate ergibt und deswegen ein mehrfaches der Dissoziationshalbwertszeit abgewartet werden muss, um das System ins Gleichgewicht zu bringen und die Qualität von Bindern zu vergleichen. In der Praxis wird deswegen häufig die Selektion unter zunehmend stringenten Bedingungen durchgeführt, was gleichzeitig meist zunehmende Abweichungen der Selektionsbedingungen von jenen Bedingungen bedeutet, unter denen der Binder letztlich seine Aufgabe erfüllen soll.

Dem Fachmann bekannte Beispiele für herkömmliche Selektionsmethoden auf Basis einer in-vitro-Selektion, mit der erforderlichen Verknüpfung eines Binders mit seiner (amplifizierbaren) genetischen Information, sind wie erwähnt das Phagedisplay mit Peptiden und scFvs (Smith, G. P., 1985, Science 228, 1315-1317; McCafferty, J, et al. 1990, Nature 34, 552-554), sowie das Selex-Verfahren für RNA-Aptamere (Ellington 1990, Gold 1990). Die Verwendung von RNA-Peptid-Fusionen beschreibt Roberts (PNAS 94, 12297). Das peptide-on-plasmid display ist in Schatz PJ, Cull MG, Martin EL, Gates CM. in Screening of peptide libraries linked to lac repressor. Methods Enzymol. 1996;267:171-191 beschrieben, und das ribosome display ist von He M, Taussig MJ (1997) in Antibody-ribosome-mRNA (ARM) complexes as efficient selection particles for in vitro display and evolution of antibody combining sites. Nucleic Acids Res 25: 5132-5134 und von Hanes J, Pluckthun A (1997) in In vitro selection and evolution of functional proteins by using ribosome display. Proc Natl Acad Sci USA 94: 4937-4942] beschrieben worden. Weitere Screening-Methoden finden sich bei Lam KS, Salmon SE, Hersh EM, Hruby VJ, Kazmierski WM, Knapp RJ (1991) A new type of synthetic peptide library for identifying ligand-binding activity. Nature 354: 82-84), ggf. gekoppelt mit einer Identifikation durch Oligonukleotide und bei Needels MC, Jones DG, Tate EH, Heinkel GL, Kochersperger LM, Dower WJ, Barrett RW, Gallop MA (1993) Generation and screening of an oligonucleotide-encoded synthetic peptide library. Proc Natl Acad Sci USA 90: 10700-10704.

Häufig tritt das Problem auf, dass einzelsträngige Nukleinsäuren eine sehr hohe unspezifische Affinität z. B. für Proteine und Peptide haben. Bei den bekannten Anreicherungsverfahren werden dann neben den gewünschten hochspezifischen gleichzeitig auch die unerwünschten hochaffinen aber unspezifischen Moleküle angereichert. Um die daraus resultierende schwierige Anreicherung von höher affinen und spezifischen Molekülen zu umgehen, müssen häufig sehr stringente Selektionsbedingungen gewählt werden. Dazu werden beispielsweise Hochsalzbedingungen (250-1000 mM Salzkonzentration), denaturierende Agenzien (Harnstoff) oder Kompetitorpolyanionen (Heparin, tRNA, etc.) in den Bindungsreaktionen eingesetzt. Nachteil der Verwendung einer erhöhten Stringenz ist es, dass die Inkubation mit dem Zielmolekül unter unphysiologischen Bedingungen erfolgt. Viele Zielmoleküle (z.B.) Proteine verändern unter diesen Bedingungen ihre Struktur bzw. sind nicht stabil. Diese Proteine werden daher möglicherweise durch die Selektion garnicht erfasst und/oder es werden unerwünschte Moleküle aufgrund ihrer durch die Stringenz veränderten Struktur erfasst.

Eine weitere Gruppe von Zielmolekülen, die mit dem SELEX-Verfahren nicht selektiert werden können, sind Moleküle, bei denen sich auch durch die beschriebenen Methoden zur Erhöhung der Stringenz die unspezifische Affinität der einzelsträngigen Nukleinsäuren nicht reduzieren lässt. Ein Beispiel hierfür sind stark positiv geladene Moleküle. In diesen Fällen lässt sich keine Anreicherung von spezifischen Bindern durch Affinitätsselektionen erreichen.

Aufgabe der Erfindung ist es demnach, ein Verfahren zur Verfügung zu stellen, das es erlaubt, so zu selektieren, dass die unerwünschten hochaffin aber unspezifisch bindenden Moleküle eher nicht ausgewählt werden, sondern überwiegend die hochspezifisch bindenden, und ein Verfahren bereitzustellen, das nicht auf Affinitätsgleichgewichtsverteilungen beruht, sondern auch Binder isolieren kann, die in einer Bibliothek nur selten vorkommen.

Eine weitere Aufgabe der Erfindung ist es, sowohl in vitro Selektionen für spezifische Binder unter annähernd physiologischen Bedingungen zu ermöglichen, als auch spezifische Binder aus Bibliotheken von Molekülen oder Partikeln, die bereits vor der Selektion eine sehr hohe Affinität zu den Zielmolekülen aufweisen, zu isolieren.

Ein Nachteil bei den meisten beschriebenen Stringenzmethoden ist, dass die Inkubation mit dem Zielmolekül unter unphysiologischen Bedingungen erfolgt. Viele Zielmoleküle (z.B.) Proteine sind unter diesen Bedingungen nicht stabil und daher sind keine Selektionen möglich. Eine weitere Gruppe von Zielmolekülen, die z.B. für das SELEX-Verfahren nicht zugänglich sind, sind Moleküle, bei denen sich auch durch die beschriebenen Stringenzmethoden die unspezifische Affinität der einzelsträngigen Nukleinsäuren nicht reduzieren lässt. Ein Beispiel hierfür sind sehr positiv geladene Moleküle. In diesen Fällen lässt sich keine Anreicherung von spezifischen Bindern durch Affinitätsselektionen erreichen. Eine weitere Aufgabe des hier beschriebenen Verfahrens ist es also, sowohl in vitro Selektionen für Binder, z.B. spezifische Binder unter annähernd physiologischen Bedingungen zu ermöglichen, als auch Binder, z.B. spezifische Binder aus Bibliotheken mit sehr hoher Ausgangsaffinität zu den Zielmolekülen zu isolieren.

Weiterhin sind aus der nicht vorveröffentlichten internationalen Anmeldung WO02/14862 A2 derselben Anmelderin ein Verfahren und eine Vorrichtung zur Charakterisierung und/oder zum Nachweis eines Bindungskomplexes bekannt. Hierbei erfolgt die Unterscheidung molekularer Trennkräfte durch einen differentiellen Krafttest.

Es war Aufgabe der vorliegenden Erfindung, ein neues Verfahren aufzufinden, mit dem aus einer Vielzahl von Molekülen solche Moleküle, die an ein Zielmolekül spezifisch binden, in wenigen Stufen aufgefunden werden können. Außerdem war es Aufgabe der Erfindung, ein Verfahren bereitzustellen, bei dem es vermieden wird, unspezifisch gebundene Moleküle mitzuschleppen. Außerdem sollte ein Verfahren bereitgestellt werden, mit dem sehr selektiv Moleküle mit ausgewählter Bindungskraft aufgefunden werden können, wobei die Bindungskraft im Vergleich zu bekannten Molekülen ausgewählt werden kann, jeweils unter den gegebenen Versuchsbedingungen. Darüber hinaus sollte die Auswahl unter solchen Bedingungen erfolgen, unter denen das ausgewählte Molekül später dann seine Aktivität entfalten soll. Schließlich war es Aufgabe der Erfindung, die Selektion durchzuführen, ohne die Einstellung eines Gleichgewichts von Bindung und Dissoziation abzuwarten, bzw. ohne die thermodynamische Dissoziation abzuwarten. Dies ist v.a. bei Komplexen mit kleinen Dissoziationsraten wünschenswert. Zur Lösung dieser Aufgaben wird ein Verfahren bereitgestellt, das sich eines neuen Prinzips zur Auswahl bedient, indem die Kraft, die notwendig ist, um den Komplex zwischen einem Binder und einem Zielmolekül zu trennen, mit einem Referenzkomplex verglichen und für die Auswahl ausgenutzt wird.

Gegenstand der Erfindung ist somit ein Verfahren zur in-vitro-Selektion von mit einem Zielmolekül bindefähigen Bindern aus einer Bibliothek von Molekülen, bei dem immobilisierte Zielmoleküle mit einer Vielzahl von Molekülen, die an einen Referenzkomplex gebunden sind, in Kontakt gebracht werden, sodass Zielmoleküle mit möglichen Bindern reagieren können und eine Verkettung aus Referenzkomplex, Binder und Zielmolekül bilden können, dann nicht gebundene Komponenten abgetrennt werden und an die Verkettungen eine Kraft angelegt wird, sodass sich eine Bindung in der Verkettung löst, und die an Zielmoleküle gebundenen Binder oder deren Komplementäre identifiziert und/oder amplifiziert werden, wobei der Referenzkomplex eine Bindungskraft aufweist, die so ausgewählt wird, dass unter Anlegen einer Zugkraft die Bindungskraft des Referenzkomplexes kleiner als die Bindungskraft eines spezifisch gebundenen Moleküls ist.

Die vorliegende Erfindung beschreibt die Durchführung einer neuen Art von in-vitro-Selektion unter Verwendung eines differentiellen Krafttests, also eine Kraftselektion. Der differentielle Krafttest wird in der internationalen Patentanmeldung PCT/EP01/09206 der Anmelderin beschrieben.

Es wurde überraschenderweise gefunden, dass durch Kraftmessung an Molekülen über das Kriterium der Trennkraft eines Bindungskomplexes neue Information für die Selektion von Bindern ausgewertet werden kann und somit ein neues Kriterium zur Auswahl bietet.

Das Prinzip der vorliegenden Erfindung besteht darin, eine Population von verschiedenen Bindermolekülen, die ein Zielmolekül binden können, hinsichtlich ihrer Trennkraft gegenüber dem Zielmolekül in zwei Subpopulationen aufzuteilen. Dabei erfolgt die Separation der Binder durch einen differentiellen Kraftvergleich mit einem Referenzkomplex, der eine mittlere Trennkraft aufweist, die zwischen den Trennkräften der kraftstarken und den Trennkräften der kraftschwachen Binder liegt.

Zur Erläuterung dienen die beigefügten Zeichnungen, die folgendes zeigen (Die Figuren sind nicht maßstabsgetreu):

Figur 1 zeigt schematisch eine bevorzugte Ausführungsform des erfindungsgemäßen Verfahrens.

Figur 2 zeigt ein Bindungspotential.

Figur 3 zeigt schematisch die Veränderung des Bindungspotentials durch Anlegen einer Kraft

Figur 4 zeigt schematisch Bindungspotenziale, wie sie im Falle einer Kraftselektion auf der Basis unterschiedlicher Potenzialdifferenzen, aber gleicher Bindungsweiten aussehen können.

Figur 5 zeigt schematisch Bindungspotenziale, wie sie im Falle einer Kraftselektion auf der Basis unterschiedlicher Bindungsweiten, aber gleicher Potenzialdifferenzen aussehen können.

Figur 6 zeigt schematisch den Aufbau des Komplexes aus Aptamer und Referenzoligonucleotid aus Beispiel 2.

Figur 7 zeigt schematisch den Aufbau der Oligonucleotide aus Beispiel 2.

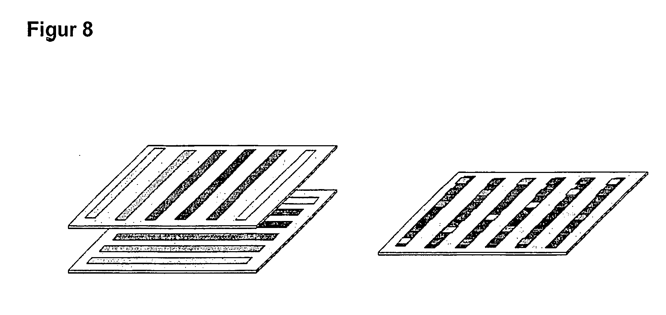

Figur 8 zeigt schematisch den Aufbau von zwei Oberflächen zur Durchführung einer Ausführungsform, die ergänzend zu dem erfindungsgemäßen Verfahren durchgeführt werden kann.

Im Gegensatz zu herkömmlichen in-vitro-Selektionsmethoden funktioniert die Kraftselektion nicht nach dem Kriterium der Affinität oder thermischer Dissoziationsraten (Off rates), sondern nach dem Kriterium der Trennkraft von Bindungskomplexen. Zur Beschreibung des Verfahrens der vorliegenden Erfindung werden die folgenden Ausdrücke wie definiert verwendet:

**Definitionen:**

| Zielmolekül: | Molekül, gegen das ein Binder gesucht wird, der dieses mit molekularer Erkennung bindet. |
|---|---|
| Binder: | Molekül, das ein Zielmolekül mit molekularer Erkennung bindet. |
| Fängermolekül | Molekül, welches mittels eines Referenzkomplexes den Binder bindet. |
| Referenzkomplex: | Komplex, dessen ausgewählte Bindungstrennkraft geringer ist als die der gesuchten Bindung molekularer Erkennung zwischen Binder und Zielmolekül; |
| | In bestimmten Ausführungsformen auch: Komplex, dessen ausgewählte Bindungstrennkraft sich von der der gesuchten Bindung molekularer Erkennung zwischen Binder und Zielmolekül unterscheidet. |
| Verkettung | Anordnung, bestehend aus einem Fängermolekül, das mittels eines Referenzkomplexes den Binder bindet, welcher Binder wiederum mittels eines Zielkomplexes das Zielmolekül bindet. |
| Zielkomplex: | Komplex, basierend auf der gesuchten Bindung molekularer Erkennung zwischen Binder und Zielmolekül |
| Kopplung | Verbindung einer Verkettung mit den Kraftangriffsmitteln (z.B. Oberflächen) |
| Nicht-Binder | Binderkandidaten, die keine Erkennungsstelle für das Zielmolekül |

aufweisen oder nicht koppeln konnten (da sie z.B. sterisch gehindert waren) und daher keinen Zielkomplex ausbilden konnten

Im Gegensatz zu bisherigen Verfahren zur Selektion von Bindern wird im hier beschriebenen Verfahren der Selektion nach Trennkraft nicht die Gleichgewichtskonstante K_g (auch Affinität genannt) oder die Dissoziationsrate (= Off rate, k_off) als Selektionskriterium genutzt, sondern die Trennkraft (F*). Der Zusammenhang zwischen Trennkraft und Dissoziationsrate ist durch Gleichung 1 gegeben, der zwischen Dissoziationsrate k_off und Gleichgewichtskonstante K_g durch Gleichung 2.

$$F^* = \frac{kT}{x_b} \cdot \left\{ \ln\left(\frac{df}{dt}\, x_b\right) - \ln\left(kTk_{off}\right) \right\} \qquad \text{Gleichung (1)}$$

wobei

| F* | Trennkraft |
|---|---|
| f | angelegte Kraft |
| k_off | thermische Off-rate (d.h. Off-rate ohne äußere angelegte Kraft) |
| x_b | Bindungsweite |
| df/dt | Kraftladungsrate |

$$K_g = \frac{k_{off}}{k_{on}} \qquad \text{Gleichung 2}$$

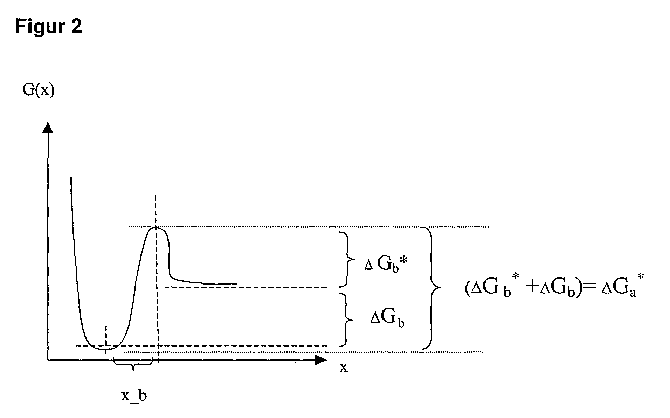

Dabei ist k_on die Assoziationsrate (= On rate).
Des weiteren gelten folgende Zusammenhänge (vgl. Figur 2):

$$k_{on} \propto \exp\left(-\frac{\Delta G_b^*}{kT}\right)$$

$$k_{off} \propto \exp\left(-\frac{\Delta G_b{}^* + \Delta G_b}{kT}\right)$$

$$K_g = \frac{k_{off}}{k_{on}} \propto \exp\left(\frac{-\Delta G_b}{kT}\right)$$

($\propto$ bedeutet: proportional zu)

Der wesentliche Unterschied zu bisherigen Verfahren besteht darin, dass die Messung der Trennkraft sowohl Informationen über die Dissoziationsrate k_off, als auch über die in Figur 2 erläuterte Bindungsweite x_b enthält, so dass unterschiedliche Binder nicht nur anhand ihrer unterschiedlichen Dissoziationsraten, sondern auch anhand ihrer unterschiedlichen Bindungsweiten unterschieden werden können. Dies ist insbesondere dann von Vorteil, wenn sich Binder aufgrund ähnlicher Dissoziationsraten mit herkömmlichen Verfahren nicht oder nur unzureichend unterscheiden lassen. Da die Bindungsweite einer Bindung unabhängig von der Dissoziationsrate variieren kann, handelt es sich hier um einen zusätzlichen Parameter, der im Kraftexperiment bestimmt werden kann und der mit den bisher beschriebenen Methoden zur Selektion von Bindern nicht zugänglich ist.

Darüber hinaus hat die Selektion von Bindern aufgrund der Trennkraft (F*) gegenüber herkömmlichen Verfahren den Vorteil, dass die Trennkraft (F*) prinzipiell sehr viel schneller bestimmt werden kann, als die Gleichgewichtskonstante oder die Dissoziationsrate einer Bindung, was insbesondere bei Bindern mit kleiner Dissoziationsrate eine genauere und sehr viel schnellere Unterscheidung der unterschiedlichen Binder zulässt.

Welche der Binder in die Subpopulation der kraftstarken Binder gelangen, hängt letztlich immer davon ab, ob ihr Zielkomplex oder ihr Referenzkomplexes unter den gewählten Versuchsbedingungen die größere Trennkraft ausweist. Diese Trennkraft resultiert zuerst aus der Art der Wechselwirkung und dem geometrischen Aufbau des jeweiligen Bindungskomplexes. Ein weiterer ausschlaggebender Parameter kann die sogenannte Kraftladungsrate sein, mit der die Zugkraft an den Komplex angelegt wird. Außerdem sind Pufferbedingungen, wie die Ionenkonzentration, der pH-Wert und die Temperatur zu berücksichtigen.

Als Kraftladungsrate bezeichnet man die Ableitung der angelegten Zugkraft nach der Zeit (df/dt). Die Kraftladungsrate resultiert aus der angelegten Zuggeschwindigkeit (v) und der Elastizität (Federkonstante k_c) des gesamten Systems, also der Verkettung von Fängermolekül, Binder und Zielmolekül inklusive der Verknüpfung mit den Kopplungspartnern, über die sie z.B. an Oberfächen gebunden sind.

Es gilt:

Zuggeschwindigkeit v = dx/dt:

$$v = \frac{dx}{dt}$$

Kraftladungsrate df/dt = v * Federkonstante k_c:

$$\frac{df}{dt} = v \cdot k_c$$

Wobei x = Strecke
f = angelegte Kraft

Die aus den intrinsischen Eigenschaften (Molekülstruktur) eines Bindungskomplexes und den äußeren Versuchsparametern (Kraftladungsrate, Puffer, Temperatur) resultierende Trennkraft eines Bindungskomplexes läßt sich am besten anhand eines Bindungspotenzials diskutieren.

Bei dieser Funktion trägt man den Abstand der beiden Bindungspartner eines Bindungskomplexes auf die x-Achse und die freie Gibbs'sche Energie (Bindungsenergie) des Bindungskomplexes auf die y-Achse auf (Figur 2). Die Potenzialdifferenz $\Delta G\_a^*$ korreliert dabei mit der thermischen Dissoziationsrate, die auch durch herkömmliche Bindetests bestimmt werden kann.

Bei der Bindungsweite x_b handelt es sich um eine zur Bindeenergie zusätzliche Dimension, die durch herkömmliche Methoden nicht bestimmt werden kann, die jedoch durch Kraftexperimente mit dem AFM oder einen differentiellen Krafttest zugänglich ist. Potenzialdifferenz $\Delta G\_a^*$ und Bindungsweite x_b sind Größen, die auch von den Versuchsbedingungen abhängig sind:

Gegenüber dem Grundzustand eines Bindungspotenzials, bei dem keine Kraft an den Bindungskomplex angelegt wird, verändert sich das effektive Potenzial mit zunehmender Kraftladungsrate.

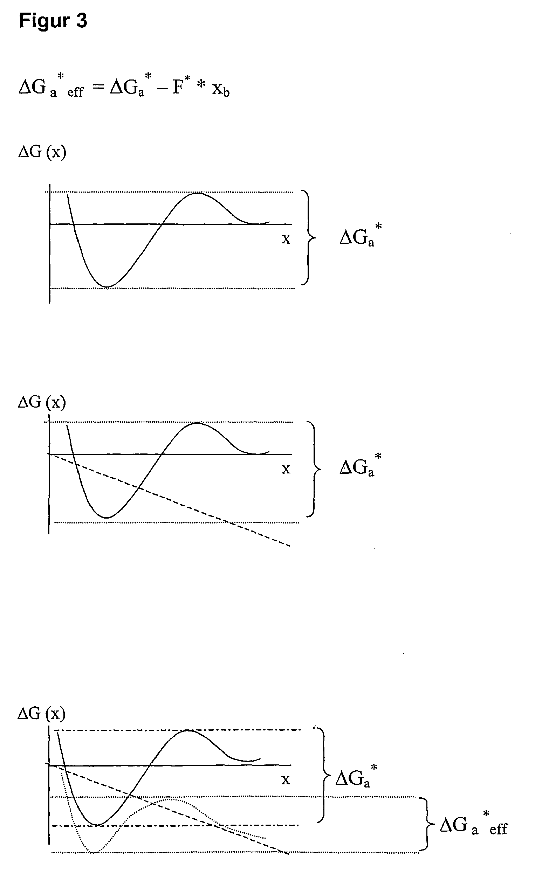

In Figur 3 wird dies durch zwei Bindungspotenziale veranschaulicht. Der Graph mit der durchgezogenen Linie steht für das Bindungspotenzial eines Komplexes im Grundzustand. Der Graph mit der gepunkteten Linie entspricht dem effektiven Potenzial des gleichen Komplexes, an den eine Kraft angelegt wird. Es ist deutlich ersichtlich, dass sich die Potenzialdifferenz $\Delta G\_a^*$ unter Kraftbeanspruchung vermindert.

Auch durch eine Variation der Ionenkonzentration kann das Potenzial einen anderen Kurvenverlauf annehmen. Insbesondere bei Komplexen aus Nukleinsäuren findet bei einer Verminderung der Natriumkonzentration (z.B. von 1M auf 50mM) eine Verkleinerung der Potenzialdifferenz $\Delta G\_a^*$ statt.

Die weiter oben beschriebene Gleichung 1 zeigt, wie Trennkraft, Bindungsweite, Dissoziationsrate, Temperatur und Kraftladungsrate formal zusammenhängen.

Für eine erfolgreiche Kraftselektion gilt also:

F*(kraftstarker Binder) > F*(Referenzkomplex) > F*(kraftschwacher Binder)

Die Separation von bindungskraftstarken und bindungskraftschwachen Bindern wird im Folgenden durch die Betrachtung zweier extremer Fälle veranschaulicht: Der Selektion nach großen Potenzialdifferenzen bei gleichen Bindungsweiten und der Selektion nach kleinen Bindungsweiten bei gleichen Potenzialdifferenzen.

**Die Separation in zwei Subpopulationen nach Potenzialdifferenzen**

Da eine große Aktivierungsenergie für die Trennung (Potenzialdifferenz $\Delta G\_a^*$) mit geringen Dissoziationsraten korreliert, kann die Kraftselektion dazu genutzt werden, Binder mit kleinen Dissoziationsraten zu selektieren (vgl. Gleichung 1).

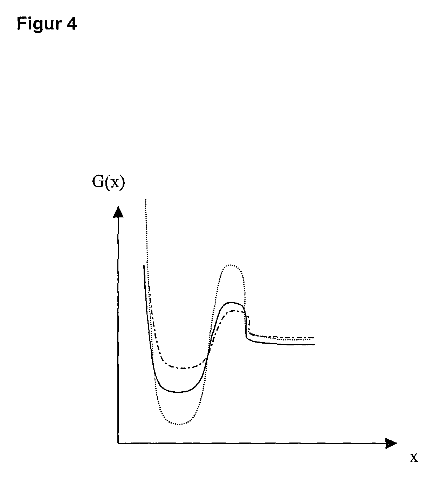

In Figur 4 wird die Separation der bindungskraftschwachen Binder mit geringer Potenzialdifferenz $\Delta G\_a^*$ (punktiert-gestrichelt) von den bindungskraftstarken Bindern mit großer Potenzialdifferenz $\Delta G\_a^*$ (punktiert) durch einen Referenzkomplex mit mittlerer Potenzialdifferenz $\Delta G\_a^*$ veranschaulicht.

**Die Trennung in zwei Subpopulationen nach Bindungsweiten**

Die Kraftselektion nach Bindungsweiten kann ggf. dazu genutzt werden, um spezifisch bindende Binder von unspezifisch bindenden zu separieren. Dabei kann man sich zu Nutze machen, dass unspezifische Bindungen i.d.R. über eine große Fläche und nicht über eine kleine, eingetiefte Bindungstasche vermittelt werden. Unspezifische Wechselwirkungen tendieren deshalb zu großen Bindungsweiten.

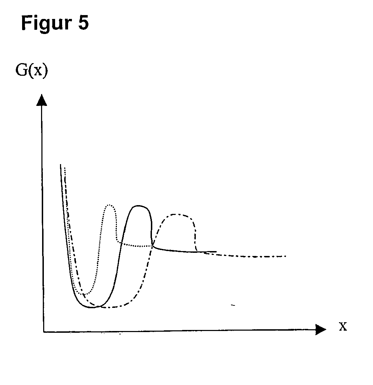

In Figur 5 wird die Separation der kraftschwachen Binder mit großer Bindungsweite (punktiert-gestrichelt) von den kraftstarken Bindern mit geringer Bindungsweite (punktiert) durch einen Referenzkomplex mit mittlerer Bindungsweite veranschaulicht.

**Kopplung und Verkettung**

Die zeitliche Reihenfolge, mit denen die Bindungskomplexe ausgebildet und die Enden der Verkettung mit den Oberflächen verbunden werden, kann variieren.

Die später in der Ausführung beschriebene Variante sieht vor, zuerst den Referenzoligo (= Fängeroligo) und das Zielmolekül zu immobilisieren, dann den Referenzkomplex ausbilden, und zum Schluß den Probenkomplex.

Genau so gut kann zuerst der Probenkomplex zwischen dem Binder und dem immobilisierten Zielmolekül ausgebildet werden, um dann beim Kontakt der Oberflächen den Referenzkomplex zu bilden. Diese Reihenfolge hat den Nachteil, dass alle Binder, die keinen Referenzkomplex ausbilden, auf der Oberfläche des Zielmoleküls verbleiben und somit die wirklich kraftstarken Binder durch falsch positive Binder kontaminieren.

Alternativ ist es denkbar, den Binder mit einem freien, biotinylierten Zielmolekül zu inkubieren, den Referenzkomplex auszubilden und abschließend das Zielmolekül an die zweite Oberfläche zu binden (Kopplung).

**Selektion bindungskraftschwacher Binder**

In der Regel sind die gesuchten Binder unter den bindungskraftstarken Molekülen zu finden. Es kann aber auch bestimmte Konstellationen geben, bei denen die gesuchten Binder in der Subpopulation zu finden sind, die mit geringerer Bindungskraft an den Zielkomplex bindet, als der Referenzkomplex. Auch für das Auffinden derartiger Binder ist das erfindungsgemäße Verfahren mit dem Selektionskriterium der Trennkraft geeignet, indem dann die entsprechend "andere" Subpopulation gewonnen wird.

**Mit dem erfindungsgemäßen Verfahren können somit gegenüber den Verfahren des Standes der Technik folgende Vorteile erreicht werden:**

Selektion spezifischer Binder bei nicht-stringenten Bedingungen.
Selektion nach neuen Eigenschaften
Selektion von Bindern unabhängig von der Einstellung eines Verteilungsgleichgewichts bzw. von der Kinetik der Dissoziation
Schnellere Selektion
Verbesserung kleiner Dissoziationsraten.
Zur Auffindung von spezifisch bindenden Paaren biologischer Moleküle wird bei bislang bekannten Verfahren, wie oben ausgeführt, häufig die Affinität als Auswahlkriterium herangezogen. Moleküle können aber für andere Moleküle aufgrund von unspezifischen Eigenschaften, wie Oberflächenladung oder hydrophobe Flächen etc. eine Affinität haben. So ist bekannt, dass RNA-Aptamere beispielsweise aufgrund der negativen Ladung ihres Zucker-Phosphatgerüsts bestimmte andere Moleküle anziehen. Antikörper und andere Proteine neigen dagegen beispielsweise zu starken, unspezifischen Wechselwirkungen mit hydrophoben Oberflächen. Im Gegensatz zu dieser unspezifischen Anlagerung ist für die spezifische Bindung das Vorliegen bestimmter Strukturmerkmale notwendig. Bei Molekülen, die zu einer spezifischen Bindung fähig sind, liegen in der Regel Bindungstaschen vor, die für die Oberfläche des Zielmoleküls eine komplementäre Struktur vorgeben. Dabei gibt es sowohl flache Kontaktregionen als auch tiefe Bindungstaschen und viele Varianten, die dazwischen liegen. Wichtig für die Affinität der Bindung ist unter anderem die Anzahl der möglichen Kontakt- und Anknüpfungspunkte. Spezifität wird dagegen durch die genaue räumliche Anordnung komplementärer Strukturelemente, verbunden mit ausreichend hohen attraktiven Wechselwirkungen, erreicht. Häufig ist die Anzahl der Kontaktpunkte in dem spezifisch bindenden Bereich um so größer, je spezifischer eine Bindung ist.

Die Trennkraft einer Bindung korreliert mit der Summe aller Wechselwirkungen, die gleichzeitig gelöst werden müssen, um einen Komplex zu trennen. Im allgemeinen besitzen Komplexe, die eine hohe Trennkraft besitzen, auch eine hohe Affinität, dagegen trifft die Umkehrung nicht allgemein zu. Im allgemeinen impliziert eine hohe Trennkraft hohe Anforderungen an die genaue räumliche Anordnung komplementärer Strukturelemente des Komplexes und läßt deswegen eine hohe Spezifität erwarten. Dies macht sich die vorliegende Erfindung zunutze, indem nicht oder je nach Ausführungsform nicht nur nach Affinität getrennt wird, sondern die zur Trennung der Bindung notwendige Kraft als unterscheidender Parameter ausgenutzt wird, der aussagekräftiger für die gewünschten Eigenschaften des Binders ist.

Entscheidend für die Kraft beim Lösen einer solchen spezifischen Bindung ist es, wie viele Bindungen oder Kontakte gleichzeitig gelöst werden müssen und welche Form die Potenzialhyperfläche - als Funktion der Ortskoordinaten der Bindungspartner und des Potenzials - hat. Dazu kommt es auf die Details der Geometrie und die Flexibilität der Komponenten an. So kann es für das Lösen einer Bindung notwendig sein, dass das System Bindungsdrehungen, Biegungen und Umorientierungen durchlaufen muss, um den angelegten Kraftvektoren auf dem Weg des geringsten Widerstandes nachzugeben. Je weniger solche Drehungen möglich sind, desto eher wird es signifikante Barrieren geben, die nur durch hohe Kraft überwunden werden können. Mit hoher Kraft gelöst werden müssen in der Regel Wasserstoffbrücken und van-der-Waals-Kontakte zwischen rigiden, molekularen Gruppen.

Erfindungsgemäß wird daher ein Verfahren zur in-vitro-Selektion von mit einem Zielmolekül bindefähigen Bindern aus einer Bibliothek von Molekülen zur Verfügung gestellt, bei dem immobilisierte Zielmoleküle mit einer Vielzahl von Molekülen, die jeweils an einen Referenzkomplex gebunden sind, in Kontakt gebracht werden, sodass Zielmoleküle mit möglichen Bindern interagieren können und eine Verkettung aus Referenzkomplex, Binder und Zielmolekül bilden können, dann an die Verkettungen eine Kraft angelegt wird, sodass sich eine Bindung in der Verkettung löst, und die an Zielmoleküle gebundenen Binder oder deren Komplementäre identifiziert und/oder amplifiziert werden, wobei der Referenzkomplex eine Bindungskraft aufweist, so dass unter Anlegen einer Zugkraft die Bindungskraft des Referenzkomplexes kleiner (oder in bestimmten Ausführungsformen: anders) als die Bindungskraft des gesuchten Moleküls ist.

Es wurde gefunden, dass eine in-vitro-Selektion von Bindern möglich ist, indem ein Vergleich von Bindungskräften durchgeführt wird. Auf diese Weise ist es nicht notwendig, viele Selektionsrunden durchzuführen, sondern in kurzer Zeit kann man die interessierenden Moleküle oder Partikel herausfinden, die an den Zielmolekülen auch noch dann gebunden bleiben, wenn auf die Bindung eine bestimmte Kraft ausgeübt wird. Dazu werden Zielmoleküle und mögliche Binder in Kontakt gebracht, wobei die Zielmoleküle immobilisiert sind und die Moleküle oder Partikel der Bibliothek an Referenzkomplexe gebunden sind. Das Verfahren ist natürlich ebenso durchführbar, wenn die interessierenden Moleküle oder Partikel immobilisiert sind und die Zielmoleküle an Referenzkomplexe gebunden sind. Der Einfachheit halber wird im Folgenden die erste Variante erläutert, wobei dieselben Ausführungen gelten, wenn Bibliothek und Zielmoleküle jeweils vertauscht sind. Wenn es zu einer Bindung eines Moleküls oder Partikels an ein Zielmolekül kommt, bildet sich eine Verkettung aus Referenzkomplex, Binder und Zielmolekül. Wenn auf diese Verkettung eine Kraft ausgeübt wird, löst sich die schwächste der Bindungen. Der Referenzkomplex ist bevorzugt so aufgebaut, dass seine Bindungskraft kleiner als die Bindungskraft der spezifischen Bindung zwischen Binder und Zielmolekül ist und auch kleiner als die Immobilisierungsbindung des Zielmoleküls ist. Hat daher der gewünschte Binder an das Zielmolekül gebunden, so löst sich bei Ausüben einer Kraft auf die Verkettung die Bindung des Referenzkomplexes als schwächste Bindung und der Binder bleibt am Zielmolekül gebunden und kann dort identifiziert und/oder amplifiziert werden. Es können auch seine Komplementäre identifiziert und/oder amplifiziert werden. Hat ein Molekül oder Partikel nur unspezifisch gebunden, so löst sich die unspezifische Bindung, das Molekül bleibt am Referenzkomplex gebunden und das Zielmolekül ist wieder frei.

Die Immobilisierung der Zielmoleküle und der Bibliothek kann jeweils an einem Träger, wie Beads, Kügelchen und dergleichen, oder an einer makroskopischen Oberfläche erfolgen. In einer bevorzugten Ausführungsform werden dazu an eine erste Oberfläche Zielmoleküle gebunden und an eine zweiten Oberfläche eine Vielzahl von Molekülen über einen Referenzkomplex gebunden, wobei die beiden Oberflächen korrespondierende Flächenabschnitte aufweisen, die so in Kontakt gebracht werden, dass Zielmoleküle mit möglichen Bindern reagieren können, die Flächen dann getrennt werden und nicht gebundene Komponenten abgetrennt werden und die an Zielmoleküle gebundenen Binder oder deren Komplementäre identifiziert, amplifiziert oder gewonnen werden, wobei der Referenzkomplex aus zwei Komponenten besteht, deren Bindungskraft so ausgewählt wird, dass unter Anlegen einer Zugkraft die Bindungskraft der beiden Referenzkomponenten aneinander kleiner als die Bindungskraft zwischen dem Zielmolekül und einem spezifisch gebundenen Binder ist, aber größer als die Bindungskraft einer unspezifischen Bindung ist.

Im Gegensatz zu den bisher bekannten Verfahren wird bei dem erfindungsgemäßen Verfahren die Bindungskraft zur Selektion von Molekülen oder Partikeln bezüglich bestimmter Eigenschaften, z.B. ihrer Spezifität für ein Zielmolekül ausgewertet. Dazu werden z.B. die zu selektierenden Moleküle über einen so genannten Referenzkomplex gebunden, der bei einer bevorzugten Ausführungsform an eine Oberfläche gebunden ist. Die über den Referenzkomplex gebundenen Moleküle werden dann mit Zielmolekülen, die in einer bevorzugten Ausführungsform ebenfalls an einer Oberfläche gebunden sind, so in Kontakt gebracht, dass es bei miteinander bindefähigen Molekülen zu einer Bindung kommen kann. Es bildet sich dabei eine Verkettung aus Zielmolekül-Binder-Referenzkomplex. Ausgewertet wird dann einerseits, ob sich diese Verkettung gebildet hat - nur diese Moleküle sind für eine weitere Betrachtung interessant - und wie stark die Bindung des Zielmoleküls an den Binder ist. Dazu wird das Verhalten der Bindung Zielmolekül-Binder - auch als Zielkomplex bezeichnet - im Vergleich zum Referenzkomplex ausgewertet, indem die Verkettungen mit den gebundenen Molekülen einer Zugkraft ausgesetzt werden, d.h. auseinander gezogen werden.

Wenn sich eine Bindung zwischen Zielmolekül und bindefähigem Molekül gebildet hat, liegt eine Kette aus Zielmolekül, damit bindefähigem Molekül und Referenzkomplex vor, auf die die Zugkraft ausgeübt wird. Diese Kette wird an der schwächsten Stelle, d.h. der Bindung mit der schwächsten Bindekraft, "aufreißen". Handelt es sich bei der Bindung zwischen dem bindefähigen Molekül und dem Zielmolekül z.B. nur um eine unspezifische Bindung, die mit schwacher Trennkraft gelöst werden kann, so wird diese unspezifische Bindung sich wieder lösen. Handelt es sich jedoch z.B. um eine spezifische Bindung hoher Bindungskraft und ist diese Bindungskraft größer als die Bindungskraft des Referenzkomplexes, so muss sich der Referenzkomplex trennen. Im ersteren Fall ist nach der Trennung der Oberflächen das Zielmolekül wieder ungebunden, im letzteren Fall ist ein Molekül mit den gewünschten Eigenschaften, z.B. einer starken Bindungskraft für das Zielmolekül und/oder einer hohen Spezifität, an das Zielmolekül gebunden.

Anschließend kann der Binder beispielsweise gewonnen und/oder identifiziert und/oder amplifiziert werden. Auf diese Weise können in einem einzigen Durchgang sehr viele Moleküle oder Partikel z.B. auf ihre Spezifität für ein Zielmolekül getestet werden und nach dem Trennen und Entfernen nicht gebundener Komponenten nur die Moleküle mit hoher Spezifität für das Zielmolekül herausgegriffen werden.

Es ist bevorzugt - aber nicht Voraussetzung für die Erfindung - wenn die ausgewählten Binder replizierbar oder amplifizierbar sind, um die Aufklärung ihrer Molekularstruktur zu erleichtern.

Das erfindungsgemäße Verfahren kann überall dort eingesetzt werden, wo es um die Auswahl einer mit einem Partner spezifisch bindefähigen Substanz aus einer Vielzahl von Substanzen geht, insbesondere um eine Auswahl von biochemischen Molekülen, die an ein Zielmolekül binden. Bei dem Zielmolekül handelt es sich um ein Molekül mit einer dreidimensionalen Struktur, die mindestens eine spezifische Bindungsstelle bereitstellt. Beispiele sind Peptide und Proteine, Nucleinsäuren, Komplexe aus Aminosäuren und Kohlehydraten und generell alle als Rezeptoren oder Liganden bezeichneten Moleküle.

Zu diesen Zielmolekülen wird ein Binder gesucht, der aus einer großen Vielzahl ausgewählt werden soll. Diese Vielzahl an möglichen Bindern wird mit biochemischen Verfahren bereitgestellt, wie sie im Stand der Technik bekannt sind. Alle Verfahren, mit denen als Binder in Betracht kommende Moleküle, Molekülkomplexe, Aggregate oder andere das entsprechende Molekül enthaltende Partikel hergestellt werden können, sind für das erfindungsgemäße Verfahren geeignet. Bevorzugte Kandidaten für das erfindungsgemä-ße Verfahren sind Moleküle bzw. Partikel, die strukturelle mit genetischer Information verbinden. Als Beispiele zu nennen sind DNA, Aptamere, Phagen (Phage Display), die entsprechenden Binderkandidaten aus Ribosom-Display, Plasmid-Display, Profusion-Proteine oder mRNA-Protein-Moleküle, Bacterial oder Viral Display, die nach Bindung und Selektion amplifiziert werden können.

Die in Betracht kommenden Binderkandidaten werden in vivo oder synthetisch hergestellt und zufällig variiert, wobei die Variation im molekularen Aufbau zu unterschiedlichen räumlichen Strukturen führt. Die unterschiedliche Struktur bedingt dabei unterschiedliche Eigenschaften insbesondere im Hinblick auf die Affinität und die Bindungskraft zum Zielmolekül.

Verfahren zur Herstellung von erfindungsgemäß geeigneten Bibliotheken werden beispielsweise von R.W. Robert und J.W. Szostak in Proc. Natl. Acad. Sci. USA, Seiten 12297-12302 beschrieben. Ein Beispiel zur Herstellung von Protein-mRNA-Molekülen ist in US-A 6 258 558 angegeben. Ein Verfahren zur Erstellung einer Phage-Display-Bibliothek ist z.B. in EP-A 768 377 beschrieben.

Zur Durchführung des erfindungsgemäßen Verfahrens werden in einer Ausführungsform zwei Oberflächen bereitgestellt, wobei an einer Oberfläche Zielmoleküle immobilisiert sind, während an der anderen Oberfläche eine Bibliothek der zu selektierenden Moleküle gebunden ist. Die Zielmoleküle, in der Regel nur eine Art, werden an eine Oberfläche in an sich bekannter Weise gebunden, wobei die Bindung so sein sollte, dass sie unter den für das Verfahren angewendeten Bedingungen stabil ist und dass sie sich auch bei dem Anlegen einer Zugkraft auf die Bindungen nicht löst. Die Art der Bindung der Zielmoleküle an die Oberfläche sollte so sein, dass die spezifische Bindungsstelle des Ziels nicht beeinträchtigt wird und frei zugänglich bleibt. Bevorzugt erfolgt die Immobilisierung über funktionelle Gruppen, die an den Zielmolekülen vorgesehen sind und funktionelle Gruppen, die die Oberfläche bereitstellt. Die Oberfläche kann in geeigneter Weise funktionalisiert werden, falls dies erforderlich ist. Die Oberfläche kann beispielsweise ein Glasträger sein, der mit einer Schicht oder Beschichtung überzogen ist.

Ein weiterer Gegenstand der Offenbarung ist daher auch eine Vorrichtung.

In gleicher Weise kann die Immobilisierung an einem Träger erfolgen, indem statt an einer makroskopischen Oberfläche an einem Partikel oder ähnlichem Träger gebunden wird. Die Bindung der Zielmoleküle kann auch über brücken- oder rechenartige Moleküle, lineare oder verzweigte Spacer erfolgen.

Die Immobilisierung der vielen verschiedenen zu untersuchenden Kandidatenmoleküle erfolgt, wie für die Zielmoleküle beschrieben, bevorzugt ebenfalls an einer Oberfläche, die in gleicher Weise, wie die erste Oberfläche zur Bindung der Zielmoleküle ausgebildet sein kann. Die zweite Oberfläche zur Aufnahme der Bibliothek kann auch aus anderem Material gebildet sein. Bei makroskopischen Oberflächen sind die beiden Oberflächen bevorzugt so ausgebildet, dass mindestens eine von ihnen aus einem elastischen Material besteht oder mit einem elastischen Material überzogen ist, um den Kontakt zwischen beiden Oberflächen zu optimieren. Möglichkeiten zur Ausbildung der Oberflächen sind beispielsweise in PCT/EP01/09206 beschrieben.

Wesentlicher Bestandteil der vorliegenden Erfindung ist der die Bindung der Bibliothek vermittelnde Referenzkomplex. Der Referenzkomplex hat die Funktion, den Vergleich der Bindungskraft zu ermöglichen. Dazu wird ein Komplex verwendet, der z.B. aus zwei Komponenten - Referenzkomponenten - besteht, die sich in definierter Weise und unter Anlegen einer ausgewählten Kraft voneinander lösen können. Es kann sich dabei um ein spezifisch bindendes Paar handeln, um Nucleinsäuredoppelstränge, aber auch um Moleküle, die an einer schwachen Stelle unter Anlegen einer Kraft auseinander brechen. In einer bevorzugten Ausführungsform werden als Referenzkomplex zwei Nucleinsäurestränge verwendet. Ein Vorteil dieser Ausführungsform besteht darin, dass z.B. durch Auswahl der Länge und Auswahl der Bindungspaare die Bindungskraft der Bindung sehr gut eingestellt werden kann. Der Vergleich der Bindungskräfte verschiedener Bindungen kann dabei so erfolgen, wie in der Anmeldung der Anmelderin PCT/EP01/09206 mit dem Titel "Verfahren und Vorrichtung zur Charakterisierung und/oder zum Nachweis eines Bindekomplexes" beschrieben.

Der Referenzkomplex ist bevorzugt ein Komplex aus zwei Komponenten, die eine Bindung ausbilden, deren Bindungsaffinität groß genug ist, um die Kandidaten der zu selektierenden Bibliothek an der Oberfläche gebunden zu halten, so lange, bis der Kontakt mit dem Zielmolekül erfolgt und deren Bindungskraft bei den Versuchsbedingungen unter einem von außen angelegten Zug schwächer ist als die Kraft einer spezifischen Bindung zwischen Zielmolekül und auszuwählendem Molekül, andererseits aber größer ist als die Kraft der unspezifischen Bindung zwischen einem Molekül der Bibliothek und dem Zielmolekül. Auf diese Weise wird dann, wenn auf beide über den Referenzkomplex und eine spezifische Bindung verbundenen Partnern ein Zug ausgeübt wird, der Referenzkomplex gelöst und damit der über den Referenzkomplex gebundene Binder auf das Zielmolekül übertragen. Wenn jedoch z.B. nur eine unspezifische Bindung stattgefunden hat, so bleiben die beiden Komponenten des Referenzkomplexes gebunden und es kommt nicht zu einer Übertragung auf das Zielmolekül.

Bevorzugt werden als Referenzkomplex, wie oben ausgeführt, zwei komplementäre Nucleinsäurestränge verwendet. Die für die Immobilisierung verwendeten komplementären Nucleinsäurestränge können jeweils sowohl über das 3'- als auch das 5'-Ende gebunden werden. Die passenden Nucleinsäurestränge können je nach den Anforderungen an die Bindungskraft genau zugeschnitten werden und in Größe und Sequenz angepasst werden. Bevorzugt werden Nucleinsäuren mit etwa 10 bis 100 Nucleotiden eingesetzt. Je nach zu untersuchender spezifischer Bindung kann außerdem ausgewählt werden, ob die Nucleinsäurestränge so gebunden werden, dass ihre einzelnen Bindungspaare reißverschlußartig nacheinander oder aber gleichzeitig getrennt werden. Wenn die Bindung so erfolgt, dass beim Ausüben einer Zugkraft auf die hybridisierten Stränge die Bindungen reißverschlussartig aufgehen können, ist ein geringere Zugkraft notwendig als dann, wenn alle Basenpaare gleichzeitig getrennt werden müssen.

Eine reißverschlußartige Trennung wird wie folgt erreicht. Wenn der immobilisierte erste Nukleinsäurestrang mit seinem 3'-Ende an der Oberfläche fixiert ist, wobei das 5'-Ende unfixiert bleibt, ist der Binderkandidat an dem komplementären zweiten Strang am 5'-Ende gebunden, so dass dann, wenn auf den Binderkandidaten ein Zug wirkt, die Kraft jeweils nur auf eine Basenpaarung des Referenzkomplexes wirkt, die eine nach der anderen aufgehen können, wodurch die Stränge somit reißverschlußartig getrennt werden.

Wenn der immobilisierte erste Strang mit dem 5'-Ende immobilisiert wird, muß der Binderkandidat entsprechend am 3'-Ende des zweiten Strangs gebunden sein, um die reißverschlußartige Trennung zu bewirken. Für diese Ausführungsform ist eine relativ geringe Zugkraft zur Trennung der Stränge erforderlich und daher kommt diese Bindungsart dann zum Einsatz, wenn das Zielmolekül, für das ein Binder gesucht wird, eine nicht sehr starke spezifische Bindung ausbildet.

Ist das Zielmolekül, für das ein Binder gesucht wird, zu einer sehr starken spezifischen Bindung fähig, so wird einerseits die Länge der Nucleinsäurestränge entsprechend eingestellt und andererseits wird der Strang so immobilisiert, dass beim Zug auf das an dem auszuwählenden Molekül gebundene Ende alle Hybridbindungen gleichzeitig aufgehen müssen, um die Stränge zu lösen. Hierzu ist eine weitaus größere Zugkraft notwendig als bei der reißverschlussartigen Öffnung.

Die Bindungskraft der Nucleinsäurestränge kann nicht nur durch die Anzahl der zu trennenden Bindungen variiert werden, sondern auch durch die Auswahl der verwendeten Basen.

Das wesentliche Kriterium für die Auswahl des Referenzkomplexes ist die Bindungskraft, die zwischen der Bindungskraft der gesuchten Bindung und der unerwünschten Bindung liegen muß.

Falls die bevorzugte Ausführungsform des erfindungsgemäßen Verfahrens durchgeführt wird, bei der die Kandidaten und die Zielmoleküle an Oberflächen gebunden sind, müssen die beiden Oberflächen mit den gebundenen Molekülen so miteinander in Kontakt gebracht werden, dass eine Bindung von bindefähigen Molekülen stattfinden kann. Die Bedingungen können bei dem erfindungsgemäßen Verfahren, im Gegensatz zum bekannten Selex-Verfahren, so ausgewählt werden, dass sie den späteren Einsatzbedingungen der Binder entsprechen und so für die gesuchten Eigenschaften der Binder, z.B. Spezifität der Bindung, unter eben diesen Bedingungen optimal sind. Als Parameter, die eingestellt werden können, kommen der pH-Wert, die Oberflächenbehandlung, die Anwendung von Puffer, die Salzkonzentration etc. in Betracht. Zum Beispiel durch Beschichtung der Oberflächen kann eine unspezifische Bindung an die Oberfläche vermieden werden, was falschpositive Ergebnisse vermeiden hilft. Da das erfindungsgemäße Verfahren insbesondere dazu geeignet ist, Binder für Rezeptoren auszuwählen, die im therapeutischen und diagnostischen Bereich eingesetzt werden, werden diese Moleküle später in der Regel unter physiologischen Bedingungen eingesetzt. Daher sollte das Selektionsverfahren auch möglichst bei physiologischen Bedingungen stattfinden, d.h. bei geringer Salzkonzentration und Konzentration an Begleitstoffen, wie sie in den Zellen vorliegen.

Wichtig für die Auswahl des Referenzkomplexes und die Auswahl der Versuchsbedingungen ist auch, inwieweit die zu untersuchenden Moleküle durch eine Krafteinwirkung beeinflusst werden. Empfindliche Moleküle, deren Sekundär- und Tertiärstruktur unter Krafteinwirkung nicht sehr stabil ist, können nur in Kombination mit einem Referenzkomplex verwendet werden, für dessen Öffnung die Kraft nicht zu groß sein darf. Da für das erfindungsgemäße Verfahren unspezifische Wechselwirkungen zwischen Binder und Zielmolekül unter bestimmten Versuchsbedingungen nicht stören, da die Auswahl über den Vergleich mit der Bindungskraft des Referenzkomplexes erfolgt und nicht über die Affinität der Bindung zwischen Zielmolekül und Binder, kann mit dem erfindungsgemäßen Verfahren auch bei relativ niedrigen Salzkonzentrationen gearbeitet werden, die die Zunahme von unspezifischen Wechselwirkungen begünstigen und deswegen für Selektionsverfahren die auf Affinitätsunterschieden beruhen, weniger geeignet sind.

Falls es notwendig ist, die Bindungskraft des Referenzkomplexes sehr genau einzustellen, kann man sich zur Messung der Bindungskraft eines AFM bedienen. Die Messung der Trennkraft von Bindungen unter Zug ist genauer in der Literaturstelle E.-L. Florin, V.T. Moy and H.E. Gaub, Science 264, 415 (1994) beschrieben.

Wenn das erfindungsgemäße Verfahren durchgeführt wurde, z.B. in der Ausführungsform, dass Zielmoleküle und möglicherweise damit bindefähige Moleküle an jeweils einer Oberfläche immobilisiert wurden, die Oberflächen und damit die Moleküle miteinander in Kontakt gebracht wurden, die Oberflächen dann wieder getrennt wurden, wobei z.B. nur spezifisch gebundene Moleküle auf der Oberfläche mit den Zielmolekülen zurückbleiben sollten, kann sich als nächster Schritt die Identifizierung und/oder Amplifikation und/oder Gewinnung der zurückgebliebenen Moleküle anschließen.

Zur Identifikation können die Moleküle einer Sequenzierung unterzogen werden oder die gebundenen Moleküle können als Matrize zur Erzeugung von Komplementären dienen, die dann wiederum identifiziert oder weiterverarbeitet werden können. Weitere, dem Fachmann bekannte Verfahren der Identifikation können herangezogen werden. Zur Gewinnung dieser Moleküle ist es am einfachsten, eine Amplifikation durchzuführen, wie sie an sich bekannt ist. Dazu werden die Bindermoleküle von den Zielmolekülen abgelöst oder die Komplementäre gewonnen und, üblicherweise mit PCR, amplifiziert. Um das Verfahren weiter zu verbessern, kann diese Amplifizierung unter Mutationsbedingungen erfolgen, so dass eine neue Bibliothek entsteht, die wiederum dem erfindungsgemäßen Verfahren unterzogen werden kann. Auf diese Weise können sehr schnell in Bezug auf die Bindefähigkeit an das Zielmolekül optimierte Moleküle aufgefunden werden.

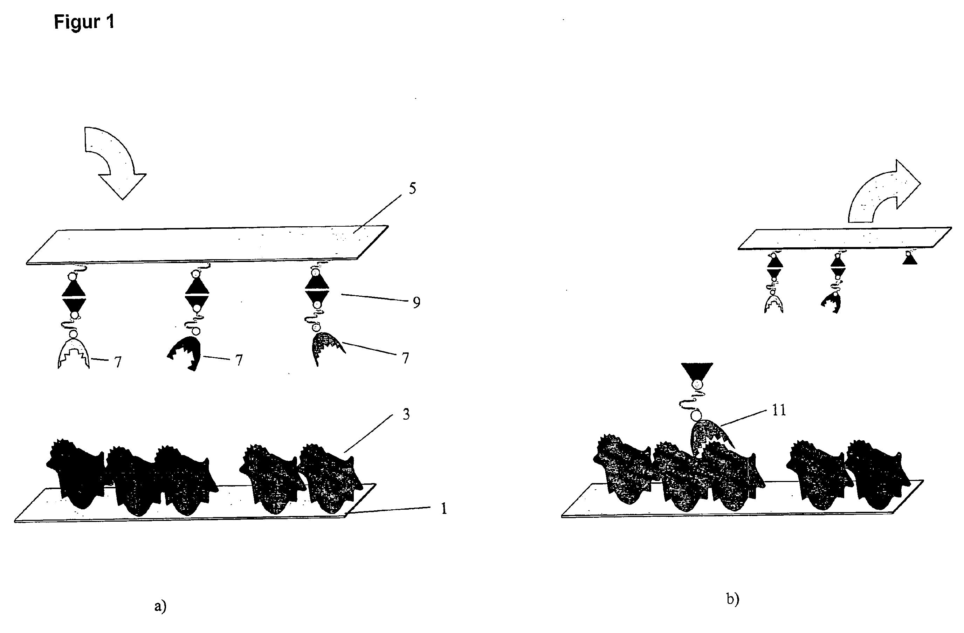

Im folgenden wird noch eine bevorzugte Ausführungsform des erfindungsgemäßen Verfahrens beschrieben, die in Figur 1 schematisch dargestellt ist.

Wie in Figur 1a) gezeigt, sind an einer Oberfläche 1 Zielmoleküle 3 immobilisiert, an einer Oberfläche 5 sind verschiedene Moleküle 7 einer Bibliothek über einen Referenzkomplex 9 gebunden. Beide Oberflächen werden so in Kontakt gebracht, dass Zielmoleküle und Moleküle der Bibliothek in Kontakt kommen und unter Bildung eines Zielkomplexes binden können. Danach wird eine Kraft auf die beiden Oberflächen ausgeübt, d.h. die beiden Oberflächen werden getrennt. Wie Figur 1b) zeigt, bleibt der Binder 11, für den die Trennkraft des Zielkomplexes größer war als die Trennkraft des Referenzkomplexes, mit dem Zielmolekül verbunden, während sich die anderen Moleküle wieder vom Zielmolekül lösen.

Nach erfolgter Trennung von Zielmolekülen und matrixgebundener Bibliothek kann der Binder auf verschiedene Art weiterbehandelt werden. Er kann identifiziert, amplifiziert und/oder sequenziert werden oder er kann als Matrize dienen zur Amplifikation von Komplementären.

Zur Veranschaulichung der Erfindung bzw. des erfinderischen Prinzips wird im Folgenden zuerst eine bestimmte Ausführung (Reihenfolge der Verkettung) einer Kraftselektion näher erörtert. Für diese Erläuterung wird die Ausführungsfrom verwendet, bei der Referenzkomplex und Zielmolekül jeweils an einer Oberfläche immobilisiert sind. Die Erfindung ist jedoch nicht auf diese Ausführungsform beschränkt, die Immobilisierung kann auch über andere Träger, z.B. Mikrokugeln, Perlen usw, die dem Fachmann bekannt sind, erfolgen. Variationen werden weiter unten diskutiert.

Man verwendet eine Bibliothek von vielen verschiedenen Binderkandidaten, die in einem Ansatz gemischt vorliegen. Die Bibliothek kann in an sich bekannter Weise hergestellt werden.

Jeder der Binderkandidaten verfügt über einen ersten Bindungspartner, der mit dem auf einer ersten Oberfläche immobilisierten Fängermolekül einen Referenzkomplex ausbildet. Außerdem verfügt jeder Binderkandidat über eine potenzielle Bindestelle, um das Zielmolekül zu binden. Das Zielmolekül wird auf einer zweiten Oberfläche immobilisiert.

Die Bibliothek mit den Binderkandiaten wird mit der ersten Oberfläche in Kontakt gebracht, damit die Binderkandidaten an das Fängermolekül binden können und somit einen Referenzkomplex ausbilden. Die erste Oberfläche wird nun mit der zweiten Oberfläche in Kontakt gebracht. Dabei kommt es zur Ausbildung eines Zielkomplexes zwischen dem Zielmolekül und solchen Binderkandidaten, deren potenzielle Bindestelle mit dem Zielmolkül wechselwirken kann. Diese Binderkandidaten werden als Binder bezeichnet. Binderkandidaten, die keine Erkennungsstelle für das Zielmolekül aufweisen oder nicht koppeln können (da sie z.B. sterisch gehindert sind) und daher keinen Zielkomplex ausbilden können, werden als Nicht-Binder bezeichnet.

Zwischen den beiden Oberflächen entsteht also eine Kopplung, bei der die beiden Oberflächen über das Fängermolekül, den Referenzkomplex, den Binder, den Zielkomplex und das Zielmolekül in einer seriellen Verkettung verbunden werden. Durch Trennen der Oberflächen kommt es zur Lösung eines Komplexes, und zwar entweder des Referenzkomplexes oder des Zielkomplexes, je nachdem, welcher der beiden Komplexe die kleinere Trennkraft aufweist.

Weist der Zielkomplex die größere Trennkraft auf, so wird der Referenzkomplex getrennt. In diesem Fall handelt es sich um einen bindungskraftstarken Binder, der beim Trennen der Oberflächen auf der zweiten Oberfläche verbleibt. Weist der Zielkomplex jedoch eine kleinere Kraftbeständigkeit als der Referenzkomplex auf, so wird der Zielkomplex getrennt und der Binder verbleibt auf der ersten Oberfläche. In diesem Fall handelt es sich um einen bindungskraftschwachen Binder. Auf diese Weise werden zwei Subpopulationen von Bindern gebildet, wobei die Subpopulation, die die gewünschten Binder enthält, weiterverarbeitet oder gewonnen werden kann. In der Regel ist dies die Population der bindungskraftstarken Binder, die auf der zweiten Oberfläche verbleiben; die bindungskraftschwachen Binder und die Nicht-Binder, die auf der ersten Oberfläche verbleiben, werden in diesem Fall verworfen.

Bei einer Kraftselektion dieser Ausführung wird also eine Population von Binderkandidaten, die aus Bindern und Nicht-Bindern besteht, in eine Subpopulation von Nicht-Bindern und kraftschwachen Bindern und eine zweite Subpopulation von kraftstarken Bindern aufgeteilt. Erfindungsgemäß erfolgt somit eine Separation der bindungskraftstarken Binder von den bindungskraftschwachen Bindern dadurch, dass die Zielkomplexe aller Binder mit dem gleichen Referenzkomplex verglichen werden und der Referenzkomplex dabei eine Trennkraft aufweist, die zwischen den Trennkräften der kraftstarken Binder und der kraftschwachen Binder liegt. Durch Vergleich der Bindungskräfte können somit gezielt Segmente von Populationen ausgewählt werden, deren Bindungskraft bei den gewählten Versuchsbedingungen in einem vorbestimmten Bereich liegt. Dies war bei den bisher bekannten Verfahren nicht möglich.

Man führt die Kraftselektion so durch, dass eine Subpopulation, in der Regel die der kraftstarken Binder, vermehrt über erwünschte Bindeeigenschaften wie z.B. erhöhte Spezifität für das Zielmolekül, verfügt.

Ein weiterer Vorteil der Erfindung besteht darin, dass man die Bedingungen, unter denen das Verfahren durchgeführt wird, gezielt so auswählen kann, dass die bindungskraftstarken Binder tatsächlich über die erwünschten Bindeeigenschaften verfügen und die bindungskraftschwachen Binder nicht, und dass man nicht auf eine Selektion durch Stringenz angewiesen ist. Es reicht zu wissen, unter welchen Selektionsbedingungen die "guten" Binder mit den erwünschten Eigenschaften eine größere Trennkraft aufweisen als die "schlechten Binder". Auch hier muss der Referenzkomplex wieder danach ausgewählt werden, dass seine Trennkraft unter diesen Selektionsbedingungen zwischen den Trennkräften der bindungskraftschwachen und der bindungskraftstarken Binder liegt.

Eine Kraftselektion erfolgt also in folgenden Schritten:

Definition der Bindeeigenschaften nach denen selektiert werden soll Auswahl von Selektionsbedingungen bei denen: Binder, die den erwünschten Bindeeigenschaften nicht entsprechen und Binder, die den erwünschten Bindeeigenschaften enstprechen, sich in der Trennkraft unterscheiden, wobei die einen eine größere Trennkraft als der Referenzkomplex und die anderen eine kleinere Trennkraft als der Referenzkomplex haben. Dazu wird ein Referenzkomplex ausgewählt, der bei den gegebenen Selektionsbedingungen eine Trennkraft aufweist, die zwischen denen der bindungskraftschwachen Binder und denen der bindungskraftstarken Binder liegt.

Das erfindungsgemäße Verfahren kann noch durch folgende Stufe ergänzt werden, bei der man die Selektivität der in einer ersten Stufe aufgefundenen Binder testen kann. Diese zusätzliche Stufe ist ebenfalls Gegenstand der vorliegenden Erfindung und kann fakulativ für alle oben beschriebenen Verfahrensvarianten angewendet werden.

Hat man Binder gegen mehrere Zielmoleküle entwickelt bzw. mit dem erfindungsgemäßen Verfahren in einer ersten Stufe aufgefunden und will diese in einem hochparallelen Assay in Chipformat verwenden, muss sichergestellt sein, dass die Binder nur "ihr" Zielmolekül binden und keine Kreuzreaktionen auftreten. Mit dem folgenden Verfahren wird dies in einem parallelen Ansatz getestet.

An einer ersten Oberfläche werden über als Kraftsensoren bezeichnete Referenzkomplexe die Binder gegen verschiede Zielmoleküle in parallelen vertikalen Streifen angebunden. An einer zweiten Oberfläche werden die Zielmoleküle in parallelen horizontalen Streifen angebunden. Der Kontakt der Oberflächen findet so statt, dass jeder Binder die Möglichkeit hat mit jedem Zielmolekül zu wechselwirken. Die Kraftsensoren weisen unter einer von außen angelegten Kraft eine geringere Bindungskraft auf als die Binder/Zielmolekül-Bindung. Binden die Binder ein Zielmolekül, bleiben sie daher beim Trennen der Oberflächen an der zweiten Oberfläche gebunden. Der Übertrag der Binder dient als Markierung der Zielmoleküle, die von den Bindern erkannt werden.

Die obige Ausführungsform des erfindungsgemäßen Verfahrens kann auch beispielsweise zur Bestimmung von Protein-Protein Wechselwirkungen verwendet werden. Um in einer Menge von Proteinen die Wechselwirkung jedes Proteins mit jedem zu testen, werden die selben Proteine einmal als Binder an die erste Oberfläche und einmal als Zielmoleküle an die zweite Oberfläche angebunden.

In einer Variante kann die oben beschriebene Ausführungsform zum Test der Selektivität von Bindepaaren ausgenutzt werden. Für Sandwichassayformate werden Paare von Bindern benötigt die gleichzeitig verschiedene Epitope eines Proteins (allg. Zielmoleküls) binden. Um zu testen, ob zwei Binder gleichzeitig das selbe Protein binden, wird der erste Binder über einen Kraftsensor an eine erste Oberfläche und der zweite Binder direkt an eine zweite Oberfläche gebunden. Der Kraftsensor als Referenzkomplex weist dabei unter einer von außen angelegten Kraft eine geringere Stabilität auf, als die im Test vorkommenden Binder/Zielmolekül-Bindungen als Zielkomplex. An den zweiten Binder wird das Protein (das Zielmolekül) gebunden. Kann das Protein (Zielmolekül) gleichzeitig von beiden Bindern gebunden werden, kann der erste Binder im Kontakt der Oberflächen das Zielmolekül binden und bleibt beim Trennen der Oberflächen an der zweiten Oberfläche gebunden. Der Übertrag des Binders signalisiert, dass das Protein als Zielmolekül gleichzeitig von beiden Binder gebunden werden kann.

Um in einem Set von Bindern, das gegen ein Protein oder eine Menge von Proteinen entwickelt wurde, diejenigen zu selektieren, die ein Sandwich-Paar bilden, werden die Binder (wie bei der Charakterisierung der Selektivität von Bindern) in parallelen vertikalen Streifen über Referenzkomplexe an eine erste Oberfläche angebunden. Die Referenzkomplexe weisen unter einer von außen angelegten Kraft eine kleinere Stabilität auf, als die im Test vorkommenden Binder/Zielmolekül-Bindungen als Zielkomplexe. An einer zweiten Oberfläche werden die gleichen Binder in parallelen horizontalen Streifen angebunden. Auf der zweiten Oberfläche wird das Protein bzw. werden die Proteine an die Binder gebunden. Der Kontakt der Oberflächen findet so statt, dass jeder Binder auf der ersten Oberfläche die Möglichkeit hat die Proteine zu binden, die auf irgendeinem Zielmolekül auf der zweiten Oberfläche gebunden sind. Kann ein Protein von zwei Bindern gleichzeitig gebunden werden, kann ein Binder auf der ersten Oberfläche im Kontakt der Oberflächen das Protein binden und bleibt beim Trennen der Oberflächen an der zweiten Oberfläche gebunden. Aus dem Muster, das sich aus dem Übertrag der Binder von der ersten auf die zweite Oberfläche ergibt, ist ersichtlich, ob zwei Binder ein Sandwichpaar bilden. Gleichzeitig lassen sich Kreuzwechselwirkungen mit anderen Proteinen erkennen. In einer weiteren Ausführungsfomr kann in einer Spalte oder Reihe die Bindungskraft der einzelnen Referenzkomplexe variiert werden, um noch genauer selektieren zu können.

Bei den erfindungsgemäßen Verfahren zum Herstellen und/oder Identifizieren von Nukleinsäureliganden aus einer Kandidatenmischung soll der Begriff der Nukleinsäureliganden solche Nukleinsäuren umfassen, die an ein Zielmolekül, bevorzugterweise ein gegebenes Zielmolekül binden. Diese Bindung erfolgt dabei typischerweise in Form einer nicht-kovalenten Bindung. Bevorzugterweise handelt es sich dabei um eine spezifische Bindung der Nukleinsäure an das Zielmolekül, insbesondere unter physiologischen Bedingungen. Die Nukleinsäure kann dabei ein Oligonukleotid oder Polynukleotid sein, wobei das Oligonukleotid so wenig wie zwei miteinander kovalent verbundene Nukleotide umfassen kann.

Der Begriff der Kandidatenmischung von Nukleinsäuren bezeichnet insgesamt eine Vielzahl von Nukleinsäuren, die im Rahmen des erfindungsgemäßen Verfahrens eingesetzt werden, um eine oder mehrere in der Mischung gegebenenfalls vorhandene Nukleinsäure(n) zu ermitteln, zu isolieren bzw. zu identifizieren, die an das Zielmolekül bindet oder binden. Eine typische Form der Kandidatenmischung stellt eine Bibliothek von Nukleinsäuremolekülen dar. Die Nukleinsäuren, wie sie in der Kandidatenmischung enthalten sind, bestehen typischerweise aus einem Segment von zusammenhängenden Nukleotiden, bevorzugterweise mindestens acht Nukleotiden, bevorzugtererweise mindestens 15 Nukleotiden, wobei die Abfolge der Nukleotide in diesem Segment zufällig ist, d.h. an einer jeden Position des entsprechenden Segmentes bzw. der Sequenz ist eine der vier Nukleobasen mit einer gewissen Wahrscheinlichkeit vorhanden. Die Wahrscheinlichkeit kann dabei für die einzelne Nukleobase von der Wahrscheinlichkeit einer oder mehreren der anderen Nukleobasen unterschiedlich sein. Durch diese zufällige Sequenz wird somit eine Anzahl von verschiedenen Nukleinsäuren definiert, die sich in der Sequenz des besagten Segmentes voneinander unterscheiden. Dabei ist es im Rahmen der vorliegenden Erfindung möglich, dass eine jede Nukleinsäure in der Kandidatenmischung auch mehrfach vorhanden sein kann.

Der Begriff des Abtrennens der mit einer größeren Bindungskraft an das Zielmolekül bindenden Nukleinsäure (n) vom Rest der Kandidatenmischung wird hierin insbesondere so verwendet, dass die Bindungskraft der Nukleinsäure (n) verschieden ist von der Bindungskraft der anderen Nukleinsäure(n) der Kandidatenmischung, entweder insgesamt oder derjenigen Nukleinsäuren der Kandidatenmischung, die grundsätzlich an das Zielmolekül binden. Dabei ist es auch im Rahmen der vorliegenden Erfindung möglich, als Referenzbindungskraft, d.h. diejenige Bindungskraft, die als Referenz herangezogen wird, einen Mittelwert der Bindungskräfte der Nukleinsäuren der Kandidatenmischung an das Zielmolekül, gegebenenfalls einen gewichteten Mittelwert, heranzuziehen. Die Gewichtung kann dabei dadurch erfolgen, dass in den Mittelwert die Anzahl der Nukleinsäuren der Kandidatenmischung einfließt, die eine bestimmte Charakteristik, beispielsweise einen bestimmten Schwellenwert, der Bindungskraft aufweisen. Der Rest der Kandidatenmischung kann dabei so viel wie die gesamten Nukleinsäuren der Kandidatenmischung minus der konkret abzutrennenden oder abgetrennten Nukleinsäure sein, aber auch so wenig wie eine einzelne abgetrennte Nukleinsäure der Kandidatenmischung. Bevorzugter Weise erfolgt die Abtrennung unter physiologischen Bedingungen, wobei physiologische Bedingungen hierin insbesondere solche Bedingungen bezeichnen, bei denen die Molarität und/oder der pH-Wert jenem Wert bzw. Werten entspricht, wie sie in zellulären biologischen Systemen auftreten oder vorherrschen. Es liegt jedoch auch im Rahmen der vorliegenden Erfindung, dass andere als physiologische Bedingungen realisiert werden zur Abtrennung der mit einer größeren Bindungskraft oder Zugkraft an das Zielmolekül bindenden Nukleinsäure vom Rest der Kandidatenmischung. Derartige andere Bedingungen können dabei Hochsalzbedingungen ebenso wie Niedrigsalzbedingungen sein. Allgemein werden im Rahmen der erfindungsgemäßen Verfahren solche Bedingungen, insbesondere Salz- und pH-Wert-Bedingungen gewählt, die gewährleisten, dass das oder die Zielmoleküle auch unter diesen Bedingungen funktionell aktiv und/oder strukturell intakt sind. Ein Beispiel für die Anwendung von Niedrigsalzbedingungen stellt die Verwendung von Kinasen dar, die beispielsweise gegenüber monovalenten Ionen empfindlich sind. Die erfindungsgemäßen Verfahren erlauben somit, dass die bei der Durchführung der Verfahren realisierten Bedingungen solche sind, wie sie vom zu untersuchenden Zielmolekül letztlich aufgrund seiner funktionellen und strukturellen Erfordernisse und nicht durch den jeweiligen Selektionsprozess erforderlich gemacht werden. In so weit erlauben die erfindungsgemäßen Verfahren, dass die für die Selektion von Nukleinsäureliganden erforderliche Stringenz nicht zulasten der funktionellen und strukturellen Erfordernisse des Zielmoleküls geht.

Bei den erfindungsgemäßen Verfahren kann dabei die Identifizierung vor oder nach der Amplifikation der jeweils an das Zielmolekül bindenden Nukleinsäure erfolgen, wobei ein jeder Schritt unabhängig vom anderen Schritt - und optional - durchgeführt werden kann. Dabei liegt es im Rahmen der vorliegenden Erfindung, dass im Rahmen der Amplifikation die Nukleinsäure hinsichtlich ihrer Sequenz, insbesondere in dem Segment mit der zufälligen Sequenz, verändert, insbesondere mutiert wird. Es ist auch im Rahmen der vorliegenden Erfindung, dass die in Schritt b) erhaltene (n) bzw. abgetrennte(n) Nukleinsäure(n) vor der Verwendung als oder im Rahmen einer Kandidatenmischung im Rahmen der erfindungsgemäßen Verfahren chemisch verändert wird. Diese chemische Änderung kann beispielsweise dadurch erfolgen, dass an einer definierten Position anstelle eines bestimmten, in der abgetrennten Sequenz vorhandenen Nukleotids mit einer gewissen Wahrscheinlichkeit ein oder mehrere der jeweils anderen Nukleotide eingebaut werden.

Dabei kann die Wahrscheinlichkeit für den Einbau eines der jeweils anderen Nukleotide gleich oder verschieden sein. Wurde beispielsweise an einer bestimmten Position der in Schritt b) abgetrennten Nukleinsäure ein Adenosin gefunden, kann die chemische Veränderung beispielsweise dadurch erfolgen, dass an eben dieser Stelle bei der chemischen Synthese mit einer bestimmten Wahrscheinlichkeit ein Guanosin, Cytosin oder Thymidin bzw. Uracil eingebaut wird.

Bei dem erfindungsgemäßen Verfahren, bei dem die mit einer höheren Kraft an das Zielmolekül bindende Nukleinsäure vom Rest der Kandidatenmischung abgetrennt wird, kann eine hinsichtlich ihrer Sekundär- und/oder Tertiärstruktur sehr spezifische Art von Nukleinsäureliganden erhalten werden, nämlich z.B. solche, die eine sehr rigide, d. h. auch starre Sekundär- und/oder Tertiärstruktur aufweisen.

Wie hierin verwendet, kann als Referenzkomplex der Komplex bestehend aus einem sogenannten Fängermolekül und bevorzugterweise einer Nukleinsäure aus der Kandidatenmischung verwendet werden. Dabei ist bevorzugt, dass alle Nukleinsäuren der Kandidatenmischung mit der gleichen Art von Fängermolekül wechselwirken. Typischerweise ist der die Wechselwirkung zwischen Fängermolekül und Nukleinsäure vermittelnde Bereich (bzw. Segment) der Nukleinsäuren verschieden von dem Segment der Nukleinsäuren mit zufälliger Sequenz, die letzten Endes für die Wechselwirkung, d. h. insbesondere die Bindung der Nukleinsäuren an das Zielmolekül verantwortlich ist, wobei dieser nicht notwendigerweise auf das Segment mit der zufälligen Sequenz beschränkt ist. Dieser Referenzkomplex weist seinerseits wiederum eine Bindungskraft im vorstehenden Sinne aus, wobei hier die Bindungskraft jene ist, die überwunden werden muss, um den Komplex aus Fängermolekül und Nukleinsäure aus der Kandidatenmischung aufzulösen bzw. die beiden Komponenten des Komplexes voneinander zu trennen.

Bei einem erfindungsgemäßen Verfahren ist dabei bevorzugterweise weiter vorgesehen, dass jene Elemente des Reaktionsansatzes, die nicht an der Kraftmessung bzw. der Ermittlung der entsprechenden Referenzkräfte beteiligt sind, im Reaktionsansatz nicht enthalten sind. Insoweit werden bevorzugterweise nicht an das Fängermolekül gebundene Nukleinsäuren der Kandidatenmischung aus dem Reaktionsansatz insbesondere nach dem Kontaktieren der Kandidatenmischung mit dem Zielmolekül entfernt.

Es liegt auch im Rahmen der Erfindung den Begriff der größeren Bindungskraft relativ zur Bindungskraft anderer oder mehrerer Nukleinsäuren der Kandidatenmischung zu verwenden. Insoweit kann es sich auch um einen vorgegebenen, d. h. definierten Wert der Bindungskraft oder der Dissoziationsrate, der in Abhängigkeit davon, welche Eigenschaften die durch die erfindungsgemäßen Verfahren selektierten Nukleinsäuren aufweisen sollen, ausgewählt wird, handeln.

Es liegt weiterhin im Rahmen der vorliegenden Erfindung, dass das Fängermolekül von der die Oberfläche ausbildenden Substanz gebildet wird, an der die Nukleinsäuren der Kandidatenmischung immobilisiert sind.

Bei den erfindungsgemäßen Verfahren ist vorgesehen, dass die erste der beiden Oberflächen, die die Nukleinsäuren der Kandidatenmischung trägt, wobei bevorzugterweise die Nukleinsäuren darauf immobilisiert sind, mit der zweiten Oberfläche, die das bzw. die Zielmolekül(e) trägt, wobei bevorzugterweise die Zielmoleküle darauf immobilisiert sind, miteinander in Kontakt gebracht wird, zumindest in dem Umfang, dass grundsätzlich eine Wechselwirkung zwischen den Nukleinsäuren der Kandidatenmischung und dem Zielmolekül bzw. einem der Zielmoleküle, die bevorzugterweise in einer Vielzahl auf der Oberfläche vorhanden sind, möglich ist. Anschließend werden die beiden Oberflächen voneinander entfernt oder getrennt. In einem anschließenden Schritt wird untersucht, welche Nukleinsäure(n) der Kandidatenmischung unter den gewählten Bedingungen an das Zielmolekül gebunden bleibt.

Es liegt dabei ebenfalls im Rahmen der vorliegenden Erfindung, dass die in einem der erfindungsgemäßen Verfahren abgetrennten Nukleinsäuren in einer weiteren Ausführung des erfindungsgemäßen Verfahrens wieder verwendet werden. In einer Ausführungsform kann insoweit die Kandidatenmischung von Nukleinsäuren aus der bzw. den Nukleinsäure(n), wie sie in den Schritten b) bzw. c) der erfindungsgemäßen Verfahren erhalten wird bzw. werden, bestehen.

Hinsichtlich der Ausgestaltung der Nukleinsäuren der Kandidatenmischung ist ergänzend auszuführen, dass diese noch weitere Segmente umfassen können, insbesondere solche, die der Amplifikation der Nukleinsäuren dienen. Bevorzugterweise stellen derartige Sequenzen Primerbindungsstellen dar, wobei bevorzugtererweise die Primerbindungsstellen der Nukleinsäuren der Kandidatenmischung gleich sind. Es kann jedoch auch vorgesehen sein, dass eine Teilpopulation der Kandidatenmischung von Nukleinsäuren eine andere Primerbindungsstelle aufweist als eine oder mehrere der Subpopulationen der Kandidatenmischung.

Im Rahmen der erfindungsgemäßen Verfahren kann eine Vorrichtung eingesetzt werden, die im Wesentlichen aus zwei einander zugewandten Oberflächen besteht, wobei auf der einen Oberfläche die Nukleinsäuren der Kandidatenmischung angeordnet sind und auf der anderen Oberfläche das immobilisierte Zielmolekül. Bevorzugterweise ist das Zielmolekül in einer Anordnung auf der Oberfläche enthalten. Diese Anordnung kann dabei regelmäßig oder unregelmäßig ausgebildet sein. Bevorzugterweise wird das Zielmolekül lediglich in einer Schicht und bevorzugterweise in einer bestimmten Orientierung auf der Oberfläche angeordnet bzw. immobilisiert sein.

Die Vorrichtung kann dabei hinsichtlich der diese aufbauenden Komponenten so ausgestaltet werden, wie diese im Zusammenhang mit der vorliegenden Beschreibung, insbesondere auch den Ansprüchen, offenbart wurden.

Weitere Merkmale, Ausführungsformen und Vorteile der erfindungsgemäßen Verfahren sowie der Verwendung der offenbarten Vorrichtung ergeben sich aus den Ansprüchen.

**Beispiel 1: Selektion von ssDNA-Bindern**

Eine Nukleinsäurebibliothek, wird durch Standardfestphasensynthese hergestellt. Die insgesamt 109nt lange Bibliothek der Binderkandidaten weist von 5' nach 3' folgende Sequenzabschnitte auf:

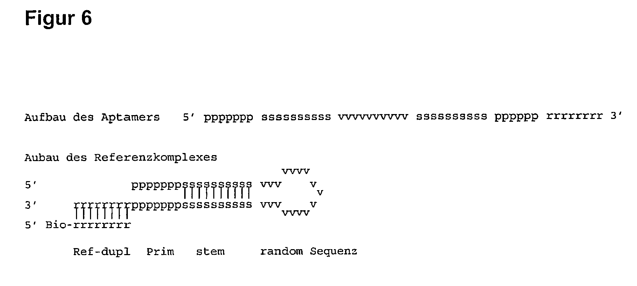

Eine konstante 18nt lange Primerannealingsequenz, die der Sequenz des Primers1 entspricht. Die mit dem Primer 1 in der späteren PCR gebildeten Nukleotidsequenz wird im folgenden als Sensestrang bezeichnet. Eine 19nt lange, konstante, GC-reiche Sequenz (stem1), zur Ausbildung eines "stem" (Stamm). Eine 20nt lange randomisierte Sequenz (Bindersequenz) zur Ausbildung einer Binderstruktur. Eine weitere konstante, 19nt lange Sequenz (stem2) die komplementär zu stem1 ist. Eine zweite 18nt lange Primerannealingsequenz die komplementär zu Primer 2 ist. Primer 2 trägt an seinem 3' Ende eine Biotingruppe. Der durch Primer in der PCR gebildete Strang wird im folgenden als Antisensestrang bezeichnet. Eine konstante 14nt lange Referenzsequenz (vgl. Figur 6). Die Basen A, T, G, C der randomisierten Sequenz sind im Verhältnis 1:1:1:1 repräsentiert.

Ein mikrostrukturierter PDMS-Stempel (PDMS = Polydimethysiloxan) wird in einem $H_2O$-Plasma aktiviert, silanisiert, und mit einem Biotin-PEG beschichtet, worauf Streptavidin angebunden wird. Die Streptavidinmoleküle werden mit einem am 5-'Ende biotinylierten Referenzoligo von 25nt Länge abgesättigt. Bei den ersten 10nt des Referenzoligos handelt es sich um einen PolyA-Spacer. Die Referenzsequenz ist (Nukleotid 11 -25) zu den Nukleotiden 85-109 der Aptamere komplementär.

Eine 5µM Lösung der Bibliothek in 5 x SSC-Puffer wird nun mit dem Stempel in Kontakt gebracht, wobei es zur Hybridisierung zwischen der Referenzsequenz des Referenzoligos und dem 3'-Terminus des Aptamers kommt. Auf dem Stempel werden in dieser Art bis zu $10^{14}$ Moleküle der Bibliothek immobilisiert.

Auf einem silanisierten Objektträger wird Carboxy-PEG angebunden. Die Carboxygruppen des PEG werden amit EDC/NHS aktiviert. Proteinantigen wird als das Zielmolekül angebunden.

Der Stempel wird nun unter PBS-Puffer mit 5mM $MgCl_2$ mit dem Obejektträger in Kontakt gebracht. Dabei können solche Aptamere, deren variable Abschnitte eine geeignete Struktur ausbildet an die Zielmoleküle binden.

Die beiden Oberflächen werden anschließend voneinander getrennt, wobei es bei jeder Verkettung zum Zerreissen entweder des Zielkomplexes oder des Referenzkomplexes kommt.

Diejenigen Aptamere, bei denen der Referenzkomplex eine geringere Bindungskraft aufweist als der Zielkomplex, werden somit auf den Objektträger übertragen. Es handelt sich hierbei um die selektierten "kraftstarken" Binder.

Die Nukleinsäurebinder werden durch denaturierende Elution mit 6 M Guanidinium-HCl oder 10 mM EDTA in Puffer von dem Objektträger mit dem Zielkomplex eluiert und anschließend mit Ethanol gefällt. Die isolierte DNA wird dann in einer Standard-PCR-Reaktion in einem Volumen von 100 µl amplifiziert. Die PCR-Zyklen werden auf Amplifikation überprüft und so viele Zyklen gefahren, bis ein deutliches Amplifikat in 1µl der PCR-Reaktion in einem 2%igen EtBr-Agarosegel zu erkennen ist. Die PCR-Fragmente werden mit Streptavidinbeads inkubiert, an die der Antisensestrang durch das über den Primer 2 eingebrachte Biotin bindet. Durch Denaturierung des Doppelstrangs mit 0,5 M NaOH wird der Sensestrang isoliert und der Antisensestrang zurückgehalten. Die Lösung mit dem Sensestrang wird auf pH 7 - pH 8 neutralisiert und kann in den nächsten Selektionszyklus eingesetzt oder sequenziert werden.

**Beispiel 2: Selektion von Oligonukleotiden**

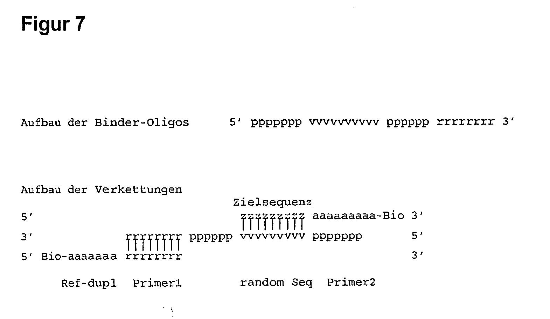

Eine Bibliothek aus DNA-Oligonukleotiden, wird synthetisiert. Die insgesamt 64 nt langen Oligos (Binderkandidaten) weisen von 5' nach 3' folgende Sequenzabschnitte auf: eine konstante 12nt lange Primer-Sequenz, die komplementär zu einem Primer (Primer1) ist. Eine 25nt lange hypervariable Sequenz (Bindersequenz) ist. Eine zweite 12nt lange Primer-Sequenz (Primer2) und eine konstante 15nt lange Referenzsequenz (vgl. Figur 7).

Ein 1 x 1 cm großer mikrostrukturierter PDMS-Stempel (PDMS = Polydimethysiloxan) wird in einem $H_2O$-Plasma aktiviert, silanisiert, und mit einem Biotin-PEG beschichtet, worauf Streptavidin angebunden wird. Die Streptavidinmoleküle werden mit einem am 5-'Ende biotinylierten Referenzoligo von 25nt Länge abgesättigt. Bei den ersten 10nt des Referenzoligos handelt es sich um einen PolyA-Spacer. Die Nukleotide 11-25 sind komplementär zum 3'-Ende der Binderkandidaten. Eine 2µM Lösung der Bibliothek in 5 x SSC-Puffer wird nun mit dem Stempel in Kontakt gebracht, wobei es zur Hybridisierung zwischen der Referenzsequenz des Referenzoligos und dem 3'-Terminus des Binderoligos kommt.

Auf einem Objektträger, auf dem kovalent Streptavidin angebunden ist, wird ein 35nt langes Zieloligo flächig angebunden. Der Stempel wird nun unter 1 x SSC -Puffer mit dem Oberjektträger in Kontakt gebracht. Dabei können Binderoligos, mit ihrer variablen Sequenz an das Zieloligo binden.

Die beiden Oberflächen werden anschließend voneinander getrennt, wobei es bei jeder Verkettung zum Zerreissen entweder des Zielkomplexes oder des Referenzkomplexes kommt.

Diejenigen Oligos, bei denen der Referenzkomplex eine geringere Bindungskraft aufweist als der Zielkomplex, werden somit auf den Objektträger übertragen. Es handelt sich hierbei um die selektierten "kraftstarken" Oligos.

Die übertragen Oligos werden bei 90°C in dd$H_2O$ von den Zieloligos abgeschmolzen. Durch eine PCR-Reaktion mit den zu den beiden Primerstellen komplementären Oligos werden die "kraftstarken" Binder amplifiziert. Durch Sequenzierung mit den selben Primern erhält man die Sequenz der kraftstarken Binderoligos.

**Patentansprüche**

1. Verfahren zur in-vitro-Selektion von mit einem Zielmolekül bindefähigen Bindern aus einer Bibliothek von Molekülen, bei dem immobilisierte Zielmoleküle mit einer Vielzahl von Molekülen, die an einen Referenzkomplex gebunden sind, in Kontakt gebracht werden, sodass Zielmoleküle mit möglichen Bindern reagieren können und eine Verkettung aus Referenzkomplex, Binder und Zielmolekül bilden können, dann nicht gebundene Komponenten abgetrennt werden und an die Verkettungen eine Kraft angelegt wird, sodass sich eine Bindung in der Verkettung löst, und die an Zielmoleküle gebundenen Binder oder deren Komplementäre identifiziert und/oder amplifiziert werden, wobei der Referenzkomplex eine Bindungskraft aufweist, die so ausgewählt wird, dass unter Anlegen einer Zugkraft die Bindungskraft der des Referenzkomplexes kleiner als die Bindungskraft eines spezifisch gebundenen Moleküls ist.

2. Verfahren zur in-vitro-Selektion von Bindern, die aufgrund einer molekularen Erkennungsstelle an ein spezielles Zielmolekül binden, aus einer Population von Molekülen, indem mindestens zwei Subpopulationen von Molekülen gebildet werden, von denen eine Population die gesuchten Binder enthält, indem gleichzeitig bei allen Molekülen der Bibliothek die Bindungskraft der Bindung an ein Zielmolekül einem Kraftvergleich unterzogen wird, wobei Referenzkomplex, Binder und Zielmolekül in einer seriellen Verkettung verbunden sind, so dass die Bindungskraft des Zielkomplexes mit der Bindungskraft eines Referenzkomplexes verglichen wird und die eine Subpopulation die Binder enthält, die eine stärkere Bindungskraft haben als der Referenzkomplex und die andere Subpopulation die Binder enthält, die eine schwächere Bindungskraft als der Referenzkomplex haben und die bezüglich der Bindungskraft gewünschte Subpopulation ausgewählt wird.

3. Verfahren nach Anspruch 1 oder Anspruch 2 **dadurch gekennzeichnet, dass** eine Selektion nach bindungskraftstarken Molekülen durchgeführt wird.

4. Verfahren nach einem der vorhergehenden Ansprüche **dadurch gekennzeichnet, dass** die Population eine Bibliothek von Molekülen umfasst.

5. Verfahren nach einem der vorhergehenden Ansprüche, **dadurch gekennzeichnet, dass** der Referenzkomplex so ausgewählt wird, dass die Bindungskraft der beiden Referenzkomponenten aneinander auch kleiner als die Bindungskraft der Immobilisierungsbindung des Zielmoleküls ist.

6. Verfahren nach einem der vorhergehenden Ansprüche, **dadurch gekennzeichnet, dass** die Binder replizierbare oder amplifizierbare Moleküle sind.

7. Verfahren nach einem der vorhergehenden Ansprüche, **dadurch gekennzeichnet, dass** eine Bibliothek von Nukleinsäuren verwendet wird.

8. Verfahren nach einem der vorhergehenden Ansprüche, **dadurch gekennzeichnet, dass** als Nukleinsäuren DNA, RNA, PNA oder LNA verwendet werden.

9. Verfahren nach einem der vorhergehenden Ansprüche, **dadurch gekennzeichnet, dass** als Bibliothek eine Phage-Display-, Ribosomen- , Plasmid-, Bacterial, Viral Display-Bibliothek oder eine Bibliothek von Protein-mRNA-Komplexen verwendet wird.

10. Verfahren nach einem der vorhergehenden Ansprüche, **dadurch gekennzeichnet, dass** der Referenzkomplex aus zwei Nucleinsäuresträngen besteht, die zumindest teilweise komplementär sind.

11. Verfahren nach Anspruch 10, **dadurch gekennzeichnet, dass** ein einzelner Nucleinsäurestrang eine Länge von 10 bis 100 Nukleotiden aufweist.

12. Verfahren nach einem der vorhergehenden Ansprüche, **dadurch gekennzeichnet, dass** als weiterer Differenzierungsparameter die Zugrate definiert eingestellt wird.

13. Verfahren nach einem der vorhergehenden Ansprüche, **dadurch gekennzeichnet, dass** der Referenzkomplex aus einem spezifisch bindenden Paar besteht, dessen Bindungskraft zwischen der Bindungskraft einer spezifischen Bindung und der Bindungskraft einer unspezifischen Bindung zwischen Zielmolekül und Binder liegt.

14. Verfahren nach einem der vorhergehenden Ansprüche, **dadurch gekennzeichnet, dass** die Zielmoleküle an einer ersten Oberfläche gebunden sind und die Moleküle der Bibliothek an einer zweiten Oberfläche gebunden sind, wobei die Oberflächen korrespondierende Flächenabschnitte aufweisen.

15. Verfahren nach einem der Ansprüche 1 bis 13, **dadurch gekennzeichnet, dass** Zielmoleküle und Moleküle der Bibliothek an Trägern, wie Chips, Partikel oder Beads immobilisiert sind.

16. Verfahren nach Anspruch 15, **dadurch gekennzeichnet, dass** die Identifizierung der selektierten Binder durch gängige Einzelmolekültechniken wie Fluoreszenz-Korrelations-Spektroskopie oder über eine durch die angelegte Kraft bewirkte Funktionsänderung an einem Zielmolekül immobiliisierten Binders erfolgt.

17. Verfahren nach einem der vorhergehenden Ansprüche, **dadurch gekennzeichnet, dass** die in einem ersten Durchlauf ausgewählten Binder in einer zweiten Stufe selektiert werden, indem im ersten Durchlauf aufgefundene Binder an einer ersten Oberfläche in parallelen Reihen über einen Referenzkomplex immobilisert werden und Zielmoleküle an einer zweiten Oberfläche in parallelen zu den Reihen senkrechten Spalten immobilisiert werden, die Oberflächen so in Kontakt gebracht werden, dass Zielmoleküle mit möglichen Bindern reagieren können und eine Verkettung aus Referenzkomplex, Binder und Zielmolekül bilden können, dann an die Verkettungen eine Kraft angelegt wird, sodass sich eine Bindung in der Verkettung löst, und die an Zielmoleküle gebundenen Binder oder deren Komplementäre identifiziert und/oder amplifiziert werden, wobei der Referenzkomplex eine Bindungskraft aufweist, die so ausgewählt wird, dass unter Anlegen einer Zugkraft die Bindungskraft der des Referenzkomplexes verschieden von der Bindungskraft eines spezifisch gebundenen Moleküls ist.

18. Verfahren nach Anspruch 17, **dadurch gekennzeichnet, dass** in jeder Reihe jeweils eine Art von Bindern immobilisiert wird und in jeder Spalte jeweils eine Art von Zielmolekülen immobilisiert wird.

**Claims**

1. Method for in-vitro selection from a library of molecules of binders, which are capable of binding to a target molecule, wherein immobilised target molecules are brought into contact with a plurality of molecules, which are bound to a reference complex, so that target molecules can react with potential binders and can form a crosslink of reference complex, binder and target molecule, non-bound components are then separated, and a force is applied to the crosslinks, so that a bond in the crosslink is split, and the binders bound to the target molecules or their complementaries are identified and/or amplified, wherein the reference complex provides a binding force, which is selected in such a manner that, subject to the application of a tensile force, the binding force of the reference complex is smaller than the binding force of a specifically-bound molecule.

2. Method for in-vitro selection from a population of molecules of binders, which bind to a specific target molecule on the basis of a molecular recognition site, in that at least two sub-populations of molecules are formed, of which one population contains the sought binders, in that, at the same time, for all molecules of the library, the binding force of the bond to a target molecule is subjected to a comparison of force, wherein reference complex, binder and target molecule are connected in a serial crosslink, so that the binding force of the target complex is compared with the binding force of the reference complex, and the one sub-population contains the binders, which have a stronger binding force than the reference complex, and the other sub-population contains the binders, which have a weaker binding force than the reference complex, and the sub-population required is selected with reference to the binding force.

3. Method according to claim 1 or claim 2,
**characterised in that**
a selection of molecules with a strong binding force is implemented.

4. Method according to any one of the preceding claims,
**characterised in that**
the population comprises a library of molecules.

**5.** Method according to any one of the preceding claims, **characterised in that** the reference complex is selected in such a manner that the binding force of the two reference components relative to one another is also smaller than the binding force of the immobilisation bond of the target molecule.

**6.** Method according to any one of the preceding claims, **characterised in that** the binders are replicable or amplifiable molecules.

**7.** Method according to any one of the preceding claims, **characterised in that** a library of nucleic acids is used.

**8.** Method according to any one of the preceding claims, **characterised in that** DNA, RNA, PNA or LNA are used as the nucleic acids.

**9.** Method according to any one of the preceding claims, **characterised in that** a phage-display library, ribosome-display library, plasmid-display library, bacterial-display library, viral-display library or a library of protein-mRNA-complexes is used as the library.

**10.** Method according to any one of the preceding claims, **characterised in that** the reference complex consists of two nucleic acid strands, which are at least partially complementary.

**11.** Method according to claim 10, **characterised in that** a single nucleic acid strand provides a length of 10 to 100 nucleotides.

**12.** Method according to any one of the preceding claims, **characterised in that** the tensile rate is used in a defined manner as a further differentiation parameter.

**13.** Method according to any one of the preceding claims, **characterised in that** the reference complex consists of a specifically-binding pair, of which the binding force is disposed between the binding force of a specific bond and the binding force of a non-specific bond between target molecule and binder.

**14.** Method according to any one of the preceding claims, **characterised in that** the target molecules are bound to a first surface, and the molecules of the library are bound to a second surface, wherein the surfaces provide portions of corresponding area.

**15.** Method according to any one of claims 1 to 13, **characterised in that** target molecules and molecules of the library are immobilised on carriers, such as chips, particles or beads.

**16.** Method according to claim 15, **characterised in that** the identification of the selected binders is implemented using current single-molecule techniques such as fluorescence-correlation spectroscopy or via a functional change of binder immobilised on a target-molecule brought about by the force applied.

**17.** Method according to any one of the preceding claims, **characterised in that** the binders selected in a first run are selected in a second stage, **in that** binders found in the first run are immobilised on a first surface in parallel rows via a reference complex, that target molecules are immobilised on a second surface in parallel columns perpendicular to the rows, that the surfaces are brought into contact in such a manner that target molecules can react with potential binders and can form a crosslink of reference complex, binder and target molecule, and that a force is then applied to the crosslink, so that a bond in the crosslink is split, and the binders bound to target molecules or their complementaries are identified and/or amplified, wherein the reference complex provides a binding force, which is selected in such a manner that, subject to the application of a tensile force, the binding force of the reference complex differs from the binding force of a specifically-bound molecule.

18. Method according to claim 17,
**characterised in that**
one type of binder is immobilised respectively in each row, and one type of target molecule is immobilised respectively in each column.

**Revendications**

1. Procédé de sélection in vitro de liants capables de se lier à une molécule ciblée ou molécule cible issue d'une bibliothèque de molécules, dans lequel des molécules ciblées immobilisées sont mises en contact avec une pluralité de molécules, qui sont liées à un complexe de référence, de sorte que des molécules ciblées peuvent réagir avec d'éventuels liants et peuvent former un enchaînement ou une association formée à partir du complexe de référence, du liant et de la molécule ciblée, les composants non liés en étant alors séparés et une force est appliquée aux enchaînements ou associations, de sorte qu'une liaison se rompt dans l'enchaînement ou l'association et que les liants liés à la molécule ciblée ou leurs complémentaires sont identifiés et/ou amplifiés, le complexe de référence présentant une force de liaison qui est choisie de telle façon que, lors de l'application d'une force de traction, la force de liaison du complexe de référence est inférieure à la force de liaison d'une molécule spécifiquement liée.

2. Procédé de sélection in vitro de liants qui en raison d'un site de reconnaissance moléculaire se lient à une molécule ciblée spécifique, issue d'une population de molécules, dans lequel au moins deux sous-populations de molécules sont formées, dont une population contient les liants recherchés, dans lequel pour toutes les molécules de la bibliothèque, la force de liaison de la liaison à une molécule ciblée est soumise simultanément à une comparaison, le complexe de référence, le liant et la molécule ciblée étant reliés de façon sérielle dans un enchaînement ou association, de sorte que la force de liaison du complexe ciblé est comparée à la force de liaison d'un complexe de référence et qu'une sous-population contient les liants qui ont une force de liaison plus élevée que le complexe de référence et que l'autre sous-population contient les liants qui ont une force de liaison plus faible que le complexe de référence et que la sous-population recherchée est choisie par rapport à la force de liaison.

3. Procédé selon la revendication 1 ou la revendication 2, **caractérisé en ce qu'**une sélection est réalisée d'après les molécules à force de liaison élevée.

4. Procédé selon l'une quelconque des revendications précédentes, **caractérisé en ce que** la population comprend une bibliothèque de molécules.

5. Procédé selon l'une quelconque des revendications précédentes, **caractérisé en ce que** le complexe de référence est choisi de façon à ce que la force de liaison des deux composants de référence entre eux est également plus faible que la force de liaison de la liaison d'immobilisation de la molécule ciblée.

6. Procédé selon l'une quelconque des revendications précédentes, **caractérisé en ce que** les liants sont des molécules qui peuvent être répliquées ou amplifiées.

7. Procédé selon l'une quelconque des revendications précédentes, **caractérisé en ce qu'**on utilise une bibliothèque d'acides nucléiques.

8. Procédé selon l'une quelconque des revendications précédentes, **caractérisé en ce que** de l'ADN, l'ARN, l'APN ou de l'ALN est utilisé comme acide nucléique.

9. Procédé selon l'une quelconque des revendications précédentes, **caractérisé en ce que** la bibliothèque utilisée est une bibliothèque avec présentation du phage, ribosomique, plasmidique, bactérienne, virale ou une bibliothèque de complexes de protéines-ARNm.

**10.** Procédé selon l'une quelconque des revendications précédentes, **caractérisé en ce que** le complexe de référence est constitué de deux brins d'acides nucléiques, qui sont au moins partiellement complémentaires.

**11.** Procédé selon la revendication 10, **caractérisé en ce qu'**un seul brin d'acides nucléiques présente une longueur de 10 à 100 nucléotides.

**12.** Procédé selon l'une quelconque des revendications précédentes, **caractérisé en ce que** l'importance de la traction est réglée de façon définie comme autre paramètre de différenciation.

**13.** Procédé selon l'une quelconque des revendications précédentes, **caractérisé en ce que** le complexe de référence est constitué d'une paire liant de façon spécifique, dont la force de liaison se situe entre la force de liaison d'une liaison spécifique et la force de liaison d'une liaison non spécifique entre une molécule ciblée et un liant.

**14.** Procédé selon l'une quelconque des revendications précédentes, **caractérisé en ce que** les molécules ciblées sont liées à une première surface et **en ce que** les molécules de la bibliothèque sont liées à une deuxième surface, les surfaces présentant des sections superficielles correspondantes.

**15.** Procédé selon l'une des revendications 1 à 13, **caractérisé en ce que** les molécules ciblées et les molécules de la bibliothèque sont immobilisées sur des supports, tels que des puces, des particules ou des billes.

**16.** Procédé selon la revendication 15, **caractérisé en ce que** l'identification des liants sélectionnés se fait par des techniques unimoléculaires habituelles telles que la spectroscopie de corrélation par fluorescence ou par un changement fonctionnel de liant immobilisé sur une molécule ciblée provoqué par l'application d'une force.

**17.** Procédé selon l'une quelconque des revendications précédentes, **caractérisé en ce que** les liants choisis lors d'une première session sont sélectionnés au cours d'une deuxième étape, dans laquelle les liants trouvés au cours de la première session sont immobilisés sur une première surface en lignes parallèles via un complexe de référence et des molécules ciblées sont immobilisées sur une deuxième surface en colonnes parallèles perpendiculaires à ces lignes, les surfaces étant mis en contact de sorte que les molécules ciblées puissent réagir avec d'éventuels liants et qu'un enchaînement ou association se forme à partir du complexe de référence, du liant et de la molécule ciblée, puis une force de liaison est appliquée aux enchaînements ou associations, de sorte qu'une liaison se rompt dans l'enchaînement ou association, et les liants liés à la molécule ciblée ou ses compléments sont identifiés et/ou amplifiés, le complexe de référence présentant une force de liaison qui est choisie de façon à ce que, sous l'effet d'une force de traction, la force de liaison du complexe de référence soit différente de la force de liaison d'une molécule spécifiquement liée.

**18.** Procédé selon la revendication 17, **caractérisé en ce qu'**une espèce de liants est immobilisée respectivement dans chaque ligne et **en ce qu'**une espèce de molécules ciblées est immobilisée respectivement dans chaque colonne.

**Figur 1**

a)

b)

EP 1 576 184 B1

**Figur 2**

$G(x)$

$\Delta G_b{}^*$

$(\Delta G_b{}^* + \Delta G_b) = \Delta G_a{}^*$

$\Delta G_b$

x

x_b

# Figur 3

$$\Delta G_a^{*}{}_{eff} = \Delta G_a^{*} - F^{*} * x_b$$

$\Delta G(x)$

$\Delta G_a^{*}$

$\Delta G(x)$

$\Delta G_a^{*}$

$\Delta G(x)$

$\Delta G_a^{*}$

$\Delta G_a^{*}{}_{eff}$

**Figur 4**

G(x)

x

**Figur 5**

# Figur 6

```
Aufbau des Aptamers    5' ppppppp ssssssssss vvvvvvvvvv ssssssssss pppppp rrrrrrrr 3'


Aubau des Referenzkomplexes
                                             vvvv
5'                  ppppppppsssssssssss vvv      v
                            |||||||||||           v
3'        rrrrrrrrrppppppppsssssssssss vvv      v
          |||||||||                       vvvv
5' Bio-rrrrrrrr

       Ref-dupl  Prim   stem      random Sequenz
```

# Figur 7

Aufbau der Binder-Oligos       5′ pppppppp vvvvvvvvv ppppp rrrrrrrr 3′

Aufbau der Verkettungen

                                     Zielsequenz
5′                                   zzzzzzzzz aaaaaaaaa-Bio 3′
                                     ↑↑↑↑↑↑↑↑↑
3′              rrrrrrrrr ppppp vvvvvvvvv ppppppp         5′
                ↑↑↑↑↑↑↑↑
5′ Bio-aaaaaaa rrrrrrrr                                   3′

      Ref-dupl  Primer1       random Seq  Primer2

**Figur 8**